# EUROPEAN PATENT SPECIFICATION

(11) **EP 2 089 015 B1**
(45) Date of publication and mention of the grant of the patent: **24.11.2010**
(21) Application number: 07871150.4
(22) Date of filing: 11.10.2007
(51) Int. Cl.: A61K 31/27, A61P 25/00

(54) **METHODS FOR TREATING DISRUPTIVE BEHAVIOR DISORDERS**
VERFAHREN ZUR BEHANDLUNG DISRUPTIVER VERHALTENSSTÖRUNGEN
PROCÉDÉS DE TRAITEMENT DE TROUBLES DU COMPORTEMENT PERTURBATEURS

(30) Priority: 27.10.2006 US 863137 P
(43) Date of publication of application: 19.08.2009
(73) Proprietor: Janssen Pharmaceutica, N.V., 2340 Beerse (BE)
(72) Inventor: HAAS, Magali, Corinth, New York 12822 (US)
(74) Representative: Warner, James Alexander
(86) International application number: PCT/US2007/081048
(87) International publication number: WO 2008/070277

(56) References cited:
- WO-A-01/13904

## Description

This application claims the benefit under 35 U.S.C. 119(e) of US Provisional application Serial No. 60/863,137 filed October 27, 2006.

### FIELD OF THE INVENTION

The present invention is directed to the use of certain carbamate compounds for the treatment of Disruptive Behavior Disorders, including both mono-therapy and co-therapy with at least one other psychoactive medication.

### BACKGROUND OF THE INVENTION

The Disruptive Behavior Disorders (DBDs) include Conduct Disorder and Oppositional Defiant Disorder and are a group of related disorders that are usually first diagnosed in childhood or adolescence. (DSM-IV-TR, Diagnostic and Statistical Manual of Mental Disorders, 4th Edition, Text Revision, American Psychiatric Association, 2000)

Oppositional Defiant Disorder (ODD) and Conduct Disorder (CD) are two of the most common psychiatric disorders affecting children and adolescents. Rates of ODD range from 2 to 16% depending on the nature of the population sample and methods of ascertainment. Rates of CD are in the same range.

According to DSM-IV-TM (The Diagnostic and Statistical Manual of Mental Disorders, 4th edition Text Revision (DSM-IV-TM) American Psychiatric Association, 2000) the characteristics of ODD include short temper, constant arguing with adults, defying rules, deliberately annoying others, blaming others for their own mistakes, being angry and resentful, spiteful and vindictive. In its severe form ODD is associated with extreme dysfunction and can be highly destructive to family life.

### Conduct Disorder

The Diagnostic and Statistical Manual of Mental Disorders, 4th edition Text Revision (DSM-IV-TR) describes the characteristics of CD as; a repetitive and persistent pattern of behavior in which the basic rights of others or major age-appropriate norms or rules are violated, as manifested by the presence of three or more of a range of criteria for 12 months. These include aggression to people and animals such as; bullying, threatening, starting fights, using a weapon to cause harm, being cruel to animal or people, stealing, forcing others into sexual activity, destruction of property, fire setting, deceitfulness or theft, breaking into homes or property, stealing things of value, etc., and serious violation of rules such as staying out over night when less than 13 years of age, running away from home, truant from school before age 13.

### Oppositional Defiant Disorder

Oppositional Defiant Disorder is one of the most common psychiatric disorders affecting children and adolescents. Rates of ODD range from 2 to 16% depending on the nature of the populations sampled and methods of ascertainment. The Diagnostic and Statistical Manual of Mental Disorders, 4th edition Text Revision (DSM-IV-TR) (American Psychiatric Association, 2000) defines the essential feature of ODD as a recurrent pattern of negativistic, defiant, disobedient, and hostile behavior toward authority figures that persists for at least 6 months (Criterion A) and is characterized by the frequent occurrence of at least four of the following behaviors: losing temper (Criterion A1), arguing with adults (Criterion A2), actively defying or refusing to comply with the requests or rules of adults (Criterion A3), deliberately doing things that will annoy other people (Criterion A4), blaming others for his or her own mistakes or misbehavior (Criterion A5), being touchy or easily annoyed by others (Criterion A6), being angry and resentful (Criterion A7), or being spiteful or vindictive (Criterion A8).

To qualify for ODD, the behaviors must occur more frequently than is typically observed in individuals of comparable age and developmental level and must lead to significant impairment in social, academic, or occupational functioning (Criterion B).

The diagnosis is not made if the disturbance in behavior occurs exclusively during the course of a Psychotic or Mood Disorder (Criterion C) or if criteria are met for Conduct Disorder or Antisocial Personality Disorder (in an individual over age 18 years) (DSM-IV-TR, American Psychiatric Association, 2000) Although ODD includes some of the features observed in Conduct Disorder (e.g., disobedience and opposition to authority figures), it does not include the persistent pattern of the more serious forms of behavior in which either the basic rights of others or age-appropriate societal norms or rules are violated (DSM-IV-TR, American Psychiatric Association, 2000). When the individual's pattern of behavior meets the criteria for both Conduct Disorder and ODD, the diagnosis of Conduct Disorder takes precedence and ODD is not diagnosed (DSM-IV-TR, American Psychiatric Association, 2000). ICD-10 (World Health Organization, 1992) also includes a category of oppositional defiant disorder, defined by the presence of markedly defiant, disobedient, provocative behavior in the absence of severe dissocial or aggressive acts that violate the law or the rights of others.

Several clinicians and researchers have expressed the belief that ODD is a mild form of conduct disorder ( Rutter and Shaffer, Am. Acad. Child Psychiatry 12:371-394, (1980)), but this issue has been reviewed (Quay, HC In Psychopathological Disorders of Childhood, 3rd ed. Edited by Quay HC and Werry JS New York, John Wiley & Sons (1986)) with the authors of the review concluding that there is considerable agreement across factor analytical studies in the finding that symptoms of disruptive behavior consistently aggregate in two groupings: one consists of all oppositional defiant disorder behavior plus some symptoms of mild physical aggression, such as fighting and bullying, while the other consists of covert, nonaggressive conduct disorder behavior, such as stealing, truancy, and running away. This aggregation into two groupings further enforces the idea that ODD and conduct disorder are distinct from each other.

Studies of ODD in community samples based on the use of specified criteria--mostly those of DSM-III--and standardized interviews show a prevalence of ODD between 1.7% and 9.9%, with a weighted average of 5.7%. Approximately one-third of all of the children with any disorder had a diagnosis of ODD (Anderson et al., Arch. Gen. Psychiatry 44:69-613, (1987)).

Overall, ODD is diagnosed more often in boys than in girls although this depends on the age of the child, studies of children 12 years of age or younger showing a prevalence of ODD in boys double that seen in girls (Anderson et at., Arch. Gen. Psychiatry 44:69-613 (1987) while studies of adolescents showed a higher prevalence of ODD in girls. By comparison, conduct disorder is diagnosed more often in boys in all age groups in most studies. This difference is yet further confirmation of the distinction between ODD and conduct disorder.

There is a high co morbidity between ODD and attention deficit hyperactivity disorder and also a high co morbidity between ODD and conduct disorder and some overlap is seen between ODD and separation anxiety, generalized anxiety disorder and major depressive disorder and there appears to be an association between ODD and communication disorders.

The diagnosis of ODD remains stable over time. Cantwell and Baker 1989 (Cantwell DP and Baker L, J. Am. Acad. Child Adolesc. Psychiatry Za:691-700 (1989)) did a 4-year follow-up study of a group of children who had been diagnosed as suffering from DSM-III disorders and found that ODD, together with autism and attention deficit hyperactivity disorder, was one of the most stable diagnoses. ODD also has the poorest recovery rate of all the behavioral psychiatric disorders. This underscores the need for effective treatments for persons diagnosed with ODD.

Thus, there remains a need to provide effective pharmacological treatments for Disruptive Behavior Disorders including both Conduct Disorder, Oppositional Defiant Disorder.

### SUMMARY OF THE INVENTION

The present invention is directed to a method for the treatment of Disruptive Behavior Disorder including both Conduct Disorder and Oppositional Defiant Disorder, comprising administering to a subject in need thereof a therapeutically effective amount of a composition that comprises at least one compound of Formula 1 or Formula 2: 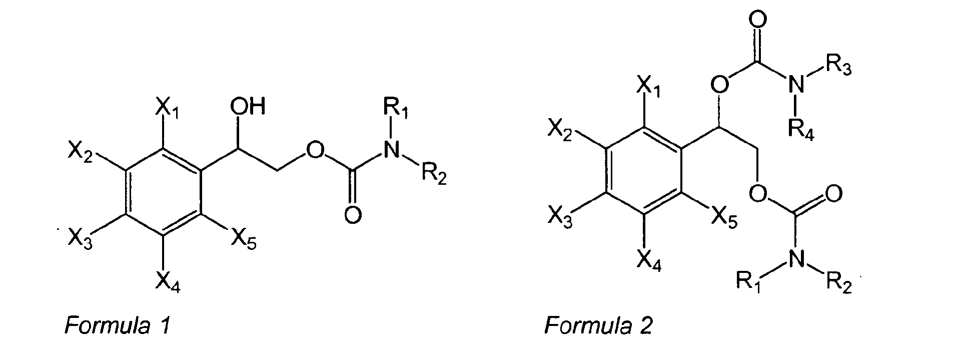 or a pharmaceutically acceptable salt or ester form thereof,
wherein
R₁, R₂, R₃ and R₄ are independently hydrogen or C₁-C₄ alkyl,
wherein
C₁-C₄alkyl is substituted or unsubstituted with phenyl, and
wherein
phenyl is substituted or unsubstituted with up to five substituents independently selected from; halogen, C₁-C₄ alkyl, C₁-C₄ alkoxy, nitro, cyano and amino
wherein amino is optionally mono or disubstituted with C₁-C₄ alkyl,
and X₁, X₂, X₃, X₄ and X₅ are independently hydrogen, fluorine, chlorine, bromine or iodine.

Embodiments of the present invention include a compound of Formula *1* or Formula 2 wherein X₁, X₂, X₃, X₄ and X₅ are independently selected from; hydrogen, fluorine, chlorine, bromine or iodine.

In certain embodiments, X₁, X₂, X₃, X₄ and X₅ are independently selected from hydrogen or chlorine.

In other embodiments, X₁ is selected from fluorine, chlorine, bromine or iodine. In another embodiment, X₁ is chlorine, and X₂, X₃, X₄ and X₅ are hydrogen. In another embodiment, R₁, R₂, R₃ and R₄ are hydrogen.

The present invention provides enantiomers of Formula 1 or Formula 2 for treating Disruptive Behavior Disorders including either Conduct Disorder ,Oppositional Defiant Disorder or DBD NOS in a subject in need thereof. In certain embodiments, a compound of Formula 1 or Formula 2 will be in the form of a single enantiomer thereof. In other embodiments, a compound of Formula 1 or Formula 2 will be in the form of an enantiomeric mixture in which one enantiomer predominates with respect to another enantiomer.

In another aspect, one enantiomer predominates in a range of from about 90% or greater. In a further aspect, one enantiomer predominates in a range of from about 98% or greater.

The present invention also provides methods comprising administering to the subject a prophylactically or therapeutically effective amount of a composition that comprises at least one compound of Formula 1 or Formula 2 wherein R₁, R₂, R₃ and R₄ are independently selected from hydrogen or C₁-C₄ alkyl; and X₁, X₂, X₃, X₄ and X₅ are independently selected from hydrogen, fluorine, chlorine, bromine or iodine.

The present invention is further directed to a method for the treatment of Disruptive Behavior Disorders including both Conduct Disorder and Oppositional Defiant Disorder comprising administering to a subject in need thereof co-therapy with a therapeutically effective amount of at least one
antidepressant and a compound of Formula 1 or Formula 2 Exemplifying the invention is a method of treating Disruptive Behavior Disorders including both Conduct Disorder and Oppositional Defiant Disorder comprising administering to a subject in need thereof a therapeutically effective amount of any of the compounds or pharmaceutical compositions described above.

In another example, the present invention is directed to a method of treating Disruptive Behavior Disorders including both Conduct Disorder and Oppositional Defiant Disorder comprising administering to a subject in need thereof at least one additional psychoactive medication in combination with any of the compounds or pharmaceutical compositions described above.

### BRIEF DESCRIPTION OF THE FIGURES

Figure 1: The DSR apparatus
Figure 2: Effect of treatment of submissive rats with Compound # 7 and fluoxetine on time spent at feeder
Figure 3: Effect of Compound # 7 and fluoxetine on dominance level in pairs of rats.
Figure 4: Effect of Treatment of Dominant Rats with Compound #7 and Lithium on Time Spend at the Feeder.
Figure 5: Effect of Compound #7 and Lithium on Dominance Level in Pairs of Rats

### DETAILED DESCRIPTION OF THE INVENTION

The present invention is directed to methods for the treatment of Disruptive Behavior Disorders including both Conduct Disorder and Oppositional Defiant Disorder comprising administering to a subject in need thereof a therapeutically effective amount of a composition containing 2-phenyl-1, 2-ethanediol monocarbomates and dicarbamates.

### The Carbamate Compounds of the Invention

Representative carbamate compounds according to the present invention include those having Formula 1 or Formula 2: 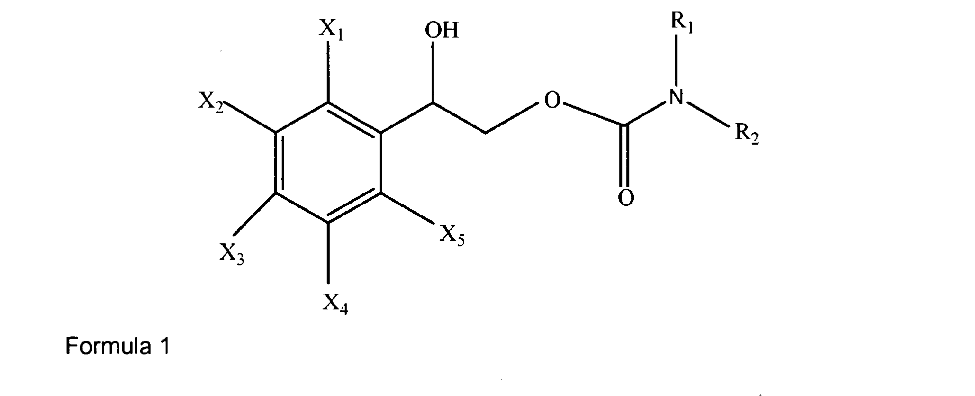 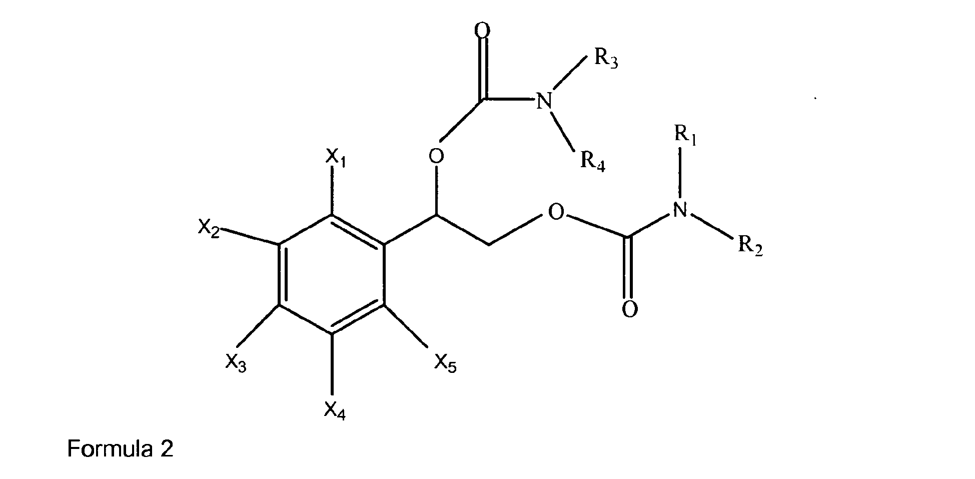 or a pharmaceutically acceptable salt or ester form thereof wherein:
R₁, R₂, R₃ and R₄ are independently hydrogen or C₁-C₄ alkyl,
   wherein
   C₁-C₄ alkyl is substituted or unsubstituted with phenyl, and
   wherein
   phenyl is substituted or unsubstituted with up to five substituents independently selected from; halogen, C₁-C₄ alkyl, C₁-C₄ alkoxy, nitro, cyano and amino
   wherein amino is optionally mono or disubstituted with C₁-C₄ alkyl,
   and X₁, X₂, X₃, X₄ and X₅ are independently hydrogen, fluorine, chlorine, bromine or iodine.

"C₁-C₄ alkyl" as used herein refers to substituted or unsubstituted aliphatic hydrocarbons having from 1 to 4 carbon atoms. Specifically included within the definition of "alkyl" are those aliphatic hydrocarbons that are optionally substituted. In a preferred embodiment of the present invention, the C₁-C₄ alkyl is either unsubstituted or substituted with phenyl.

The term "phenyl", as used herein, whether used alone or as part of another group, is defined as a substituted or unsubstituted aromatic hydrocarbon ring group having 6 carbon atoms. Specifically included within the definition of "phenyl" are those phenyl groups that are optionally substituted. For example, in a preferred embodiment of the present invention, the, "phenyl" group is either unsubstituted or substituted with halogen, C₁-C₄ alkyl, C₁-C₄ alkoxy, amino, nitro, or cyano.

In a preferred embodiment of the present invention, X₁ is fluorine, chlorine, bromine or iodine and X₂, X₃, X₄, and X₅ are hydrogen.

In another preferred embodiment of the present invention, X₁, X₂, X₃, X₄, and X₅ are, independently, chlorine or hydrogen.

In another preferred embodiment of the present invention, R₁, R₂, R₃, and R₄ are all hydrogen.

It is understood that substituents and substitution patterns on the compounds of the present invention can be selected by one of ordinary skill in the art to provide compounds that are chemically stable and that can be readily synthesized by techniques known in the art as well as the methods provided herein.

Representative 2-phenyl-1, 2-ethanediol monocarbomates and dicarbamates include, for example, the following compounds:

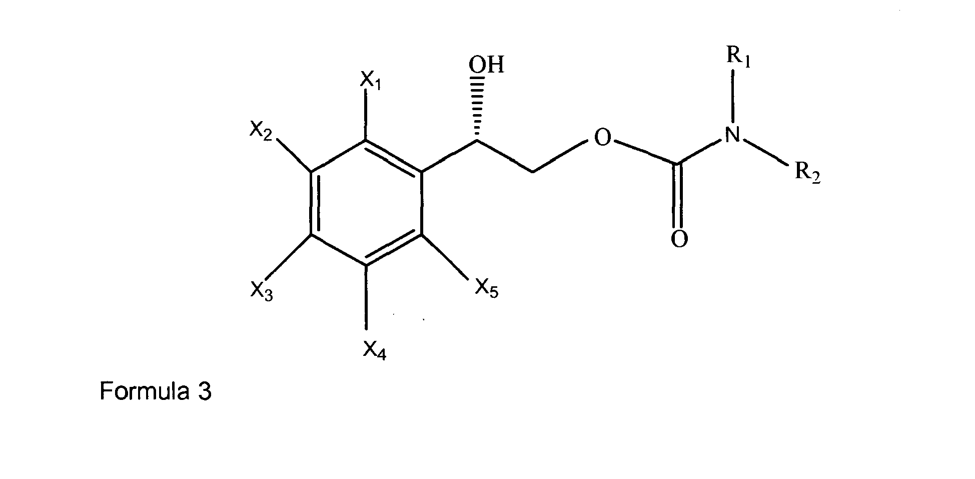

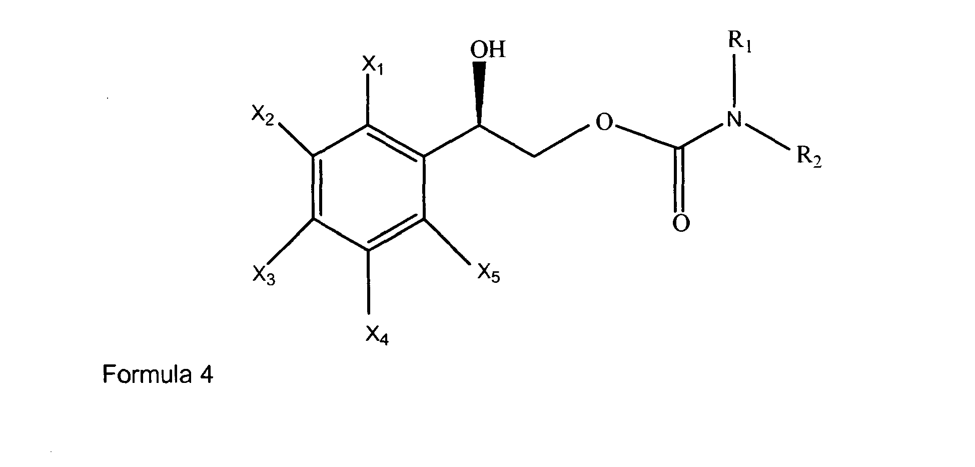

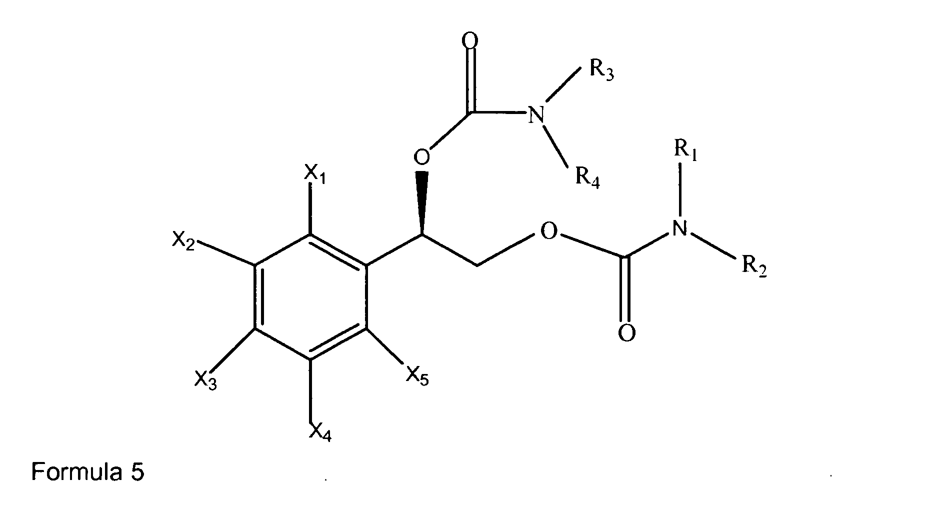

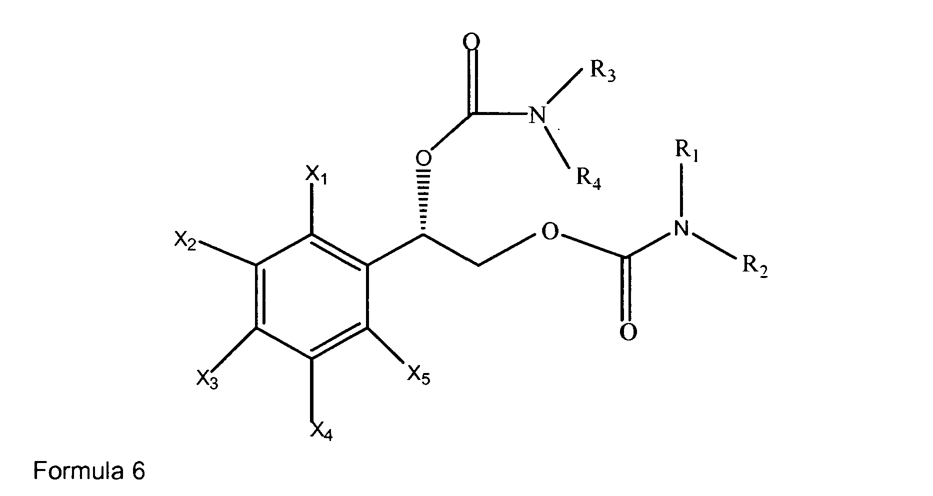

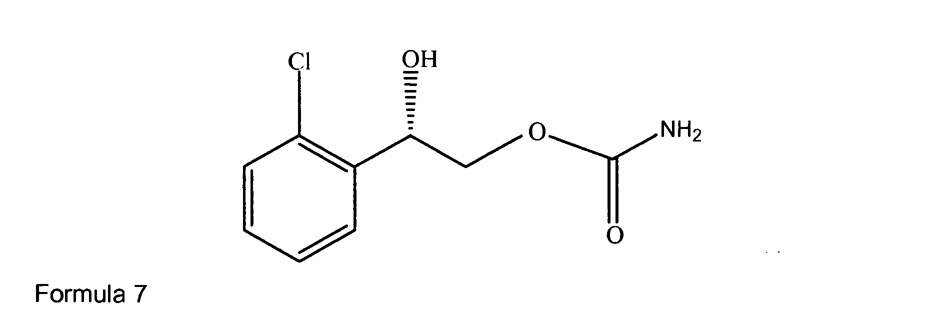

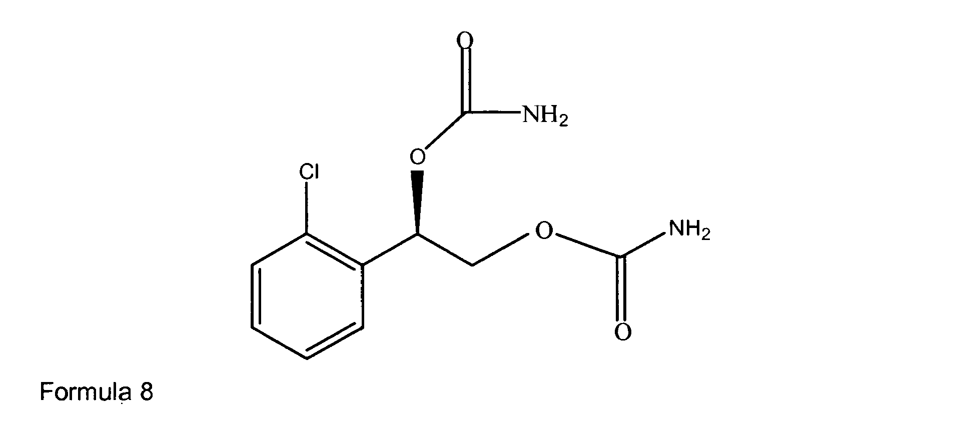

Suitable methods for synthesizing and purifying the carbamate compounds, including carbamate enantiomers, used in the methods of the present invention are well known to those skilled in the art. For example, pure enantiomeric forms and enantiomeric mixtures of 2-phenyl-1, 2-ethanediol monocarbomates and dicarbamates are described in United States Patent Numbers 5,854,283; 5,698,588, and 6,103,759 the disclosures of which are herein incorporated by reference in their entirety.

The present invention includes the use of isolated enantiomers of Formula 1 or Formula 2.

In one preferred embodiment, a pharmaceutical composition comprising the isolated S-enantiomer of Formula 1 is used to treat depression in a subject.

In another preferred embodiment, a pharmaceutical composition comprising the isolated R-enantiomer of Formula 2 is used to treat Conduct Disorder, Oppositional Defiant Disorder and Disruptive Behavior Disorder in a subject.

In another embodiment, a pharmaceutical composition comprising the isolated S-enantiomer of Formula 1 and the isolated R-enantiomer of Formula 2 can be used to treat Disruptive Behavior Disorders in a subject in need thereof.

The present invention also includes the use of mixtures of enantiomers of Formula 1 or Formula 2. In one aspect of the present invention, one enantiomer will predominate. An enantiomer that predominates in the mixture is one that is present in the mixture in an amount greater than any of the other enantiomers present in the mixture, e.g., in an amount greater than 50%. In one aspect, one enantiomer will predominate to the extent of 90% or to the extent of 91%, 92%, 93%, 94%, 95%, 96%, 97% or 98% or greater.

In one preferred embodiment, the enantiomer that predominates in a composition comprising a compound of Formula 1 is the S-enantiomer of Formula 1. In another preferred embodiment, the enantiomer that predominates in a composition comprising a compound of Formula 2 is the R-enantiomer of Formula 2.

In a preferred embodiment of the present invention, the enantiomer that is present as the sole enantiomer or as the predominate enantiomer in a composition of the present invention is represented by Formula 3 or Formula 5, wherein X₁, X₂, X₃, X₄, X₅, R₁, R₂, R₃, and R₄ are defined as above, or by Formula 7 or Formula 8.

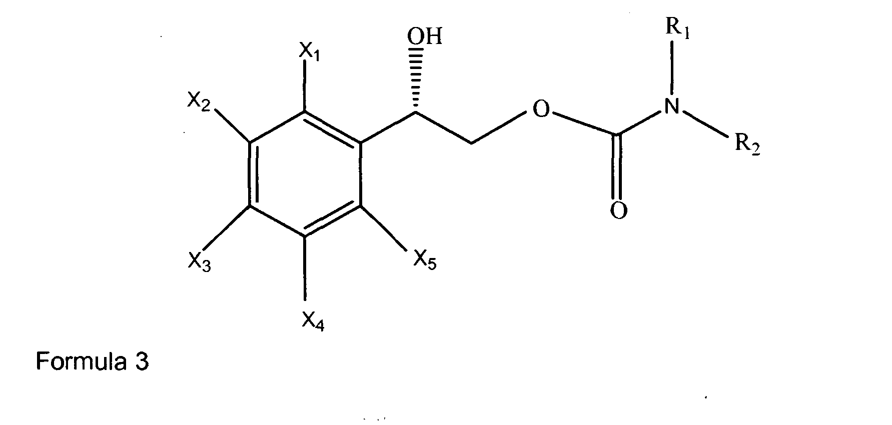

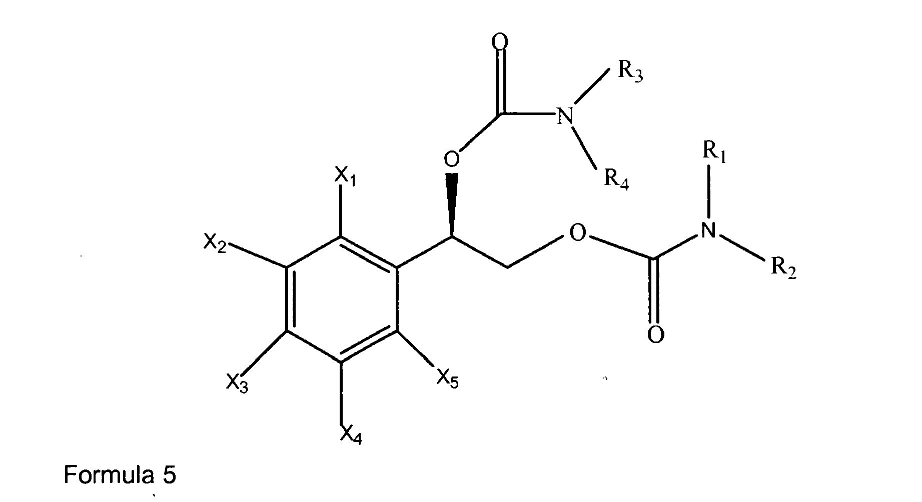

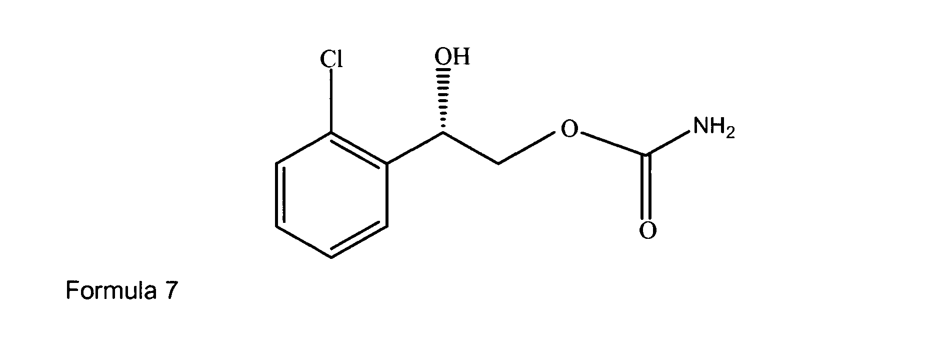

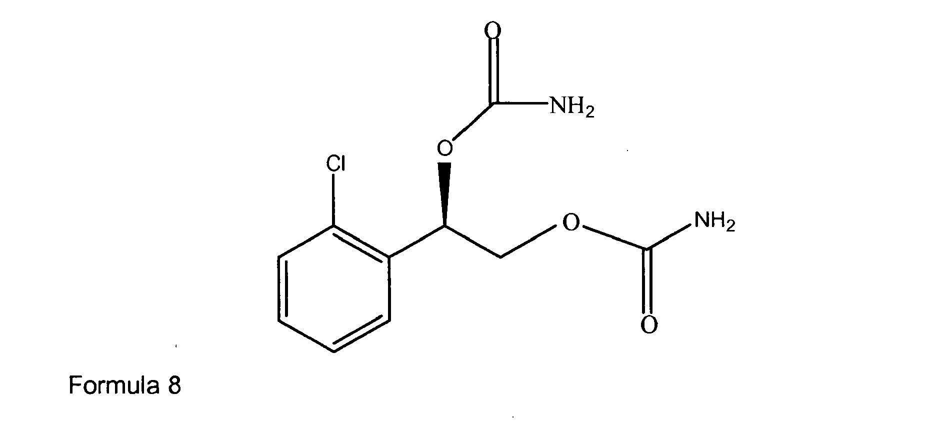

The present invention provides methods of using enantiomers and enantiomeric mixtures of compounds represented by Formula 1 and Formula 2 or a pharmaceutically acceptable salt or ester form thereof:
A carbamate enantiomer of Formula 1 or Formula 2 contains an asymmetric chiral carbon at the benzylic position, which is the aliphatic carbon adjacent to the phenyl ring.

An enantiomer that is isolated is one that is substantially free of the corresponding enantiomer. Thus, an isolated enantiomer refers to a compound that is separated via separation techniques or prepared free of the corresponding enantiomer.

The term "substantially free," as used herein, means that the compound is made up of a significantly greater proportion of one enantiomer. In preferred embodiments, the compound includes at least about 90% by weight of a preferred enantiomer.

In other embodiments of the invention, the compound includes at least about 99% by weight of a preferred enantiomer. Preferred enantiomers can be isolated from racemic mixtures by any method known to those skilled in the art, including high performance liquid chromatography (HPLC) and the formation and crystallization of chiral salts, or preferred enantiomers can be prepared by methods described herein.

Methods for the preparation of preferred enantiomers would be known to one of skill in the art and are described, for example, in Jacques, et al., Enantiomers, Racemates and Resolutions (Wiley Interscience, New York, 1981); Wilen, S.H., et al., Tetrahedron 33:2725 (1977); Eliel, E.L. Stereochemistry of Carbon Compounds (McGraw-Hill, NY, 1962); and Wilen, S.H. Tables of Resolving Agents and Optical Resolutions p. 268 (E.L. Eliel, Ed., Univ. of Notre Dame Press, Notre Dame, IN 1972).

Additionally, compounds of the present invention can be prepared as described in United States Patent Number 3,265,728 (the disclosure of which is herein incorporated by reference in its entirety and for all purposes), 3,313,692 (the disclosure of which is herein incorporated by reference in its entirety and for all purposes), and the previously referenced United States Patent Numbers 5,854,283; 5,698,588, and 6,103,759 (the disclosures of which are herein incorporated by reference in their entirety and for all purposes).

The present invention is further directed to the treatment of Disruptive Behavior Disorders including both Conduct Disorder and Oppositional Defiant Disorder comprising administering to a subject in need thereof a therapeutically effective amount of a compound of Formula 1 or Formula 2 in combination with at least one additional psychoactive medication.

As used herein, the term **"Disruptive Behavior Disorders"** or " **(DBDs)**" shall be defined to include: Conduct Disorder, Childhood-Onset Type 312.81; Conduct Disorder, Adolescent-Onset Type 312.82; Conduct Disorder, Unspecified Onset 312.89; Oppositional Defiant Disorder 313.81 and Disruptive Behavior Disorder Not Otherwise Specified 312.9 as these disorders are described in the DSM-IV-TR (The Diagnostic and Statistical Manual of Mental Disorders, 4th edition Text Revision (DSM-IV-TR) (American Psychiatric Association, 2000)

As used herein, unless otherwise noted, the term **"psychoactive medication"** shall mean any pharmaceutical agent that may be used to treat or to augment the treatment of Disruptive Behavior Disorders including both Conduct Disorder, Oppositional Defiant Disorder and Disruptive Behavior Disorders NOS. Suitable examples include, but are not limited to; stimulants, including but not limited to methylphenidate, amphetamine and modafinil, major tranquilizers, such as molindone, haloperidol and chlorpromazine, minor tranquilizers, including benzodiazepines, antidepressants, including but not limited to tricyclics such as imipramine, amitriptyline, desipramine, nortriptyline, doxepin, protriptyline, trimipramine, clomipramine, amoxapine, and the like; tetracyclics such as maprotiline, and the like; non-cyclics such as nomifensine, and the like; triazolopyridines such as trazodone, and the like; serotonin reuptake inhibitors such as fluoxetine, sertraline, paroxetine, citalopram, fluvoxamine, and the like; serotonin receptor antagonists such as nefazodone, and the like; combined serotonin-noradrenergic reuptake inhibitors such as venlafaxine, milnacipran and the like; noradrenergic and specific serotonergic agents such as mirtazapine, and the like; noradrenaline reuptake inhibitors such as reboxetine, and the like; atypical antidepressants such as bupropion, and the like; natural products such as Kava-Kava, St. John's Wort, and the like; dietary supplements such as s-adenosylmethionine, and the like, mono-amine oxidase inhibitors such as phenelzine, tranylcypromine, moclobemide, and the like;. Preferably the stimulant is selected from the group consisting of methylphenidate and modafinil. Preferably, the antidepressant is selected from the group consisting of fluoxetine, paroxetine, citalopram, fluvoxamine, bupropion, venlafaxine and sertraline.

One skilled in the art would be able to readily determined recommended dosage levels for known and / or marketed psychoactive medications including antidepressant, tranquilizers, antipsychotic drugs and stimulant drugs by consulting appropriate references such as drug package inserts, FDA guidelines, the Physician's Desk Reference, and the like.

The term **"subject"** as used herein, refers to an animal, preferably a mammal, most preferably a human, who has been the object of treatment, observation or experiment.

The term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

Wherein the present invention is directed to co-therapy or combination therapy, comprising administration of one or more compound(s) of Formula 1 or Formula 2 and one or more psychoactive medications, "therapeutically effective amount" shall mean that amount of the combination of agents taken together so that the combined effect elicits the desired biological or medicinal response. For example, the therapeutically effective amount of co-therapy comprising administration of a compound of formula (I) or formula (II) and at least on antidepressant would be the amount of the compound of formula (I) or formula (II) and the amount of the psychoactive medication that when taken together or sequentially have a combined effect that is therapeutically effective. Further, it will be recognized by one skilled in the art that in the case of co-therapy with a therapeutically effective amount, as in the example above, the amount of the compound of Formula 1 or Formula 2 and/or the amount of the psychoactive medication individually may or may not be therapeutically effective.

As used herein, the terms **"co-therapy"** and **"combination therapy"** shall mean treatment of a subject in need thereof by administering one or more compounds of Formula 1 or Formula 2 in combination with one or more additional psychoactive medication(s), wherein the compound(s) of Formula 1 or Formula 2 and the additional psychoactive medication(s) are administered by any suitable means, simultaneously, sequentially, separately or in a single pharmaceutical formulation. Where the compound(s) of Formula 1 or Formula 2 and the additional psychoactive medication(s) are administered in separate dosage forms, the number of dosages administered per day for each compound may be the same or different.

The compound(s) of Formula 1 or Formula 2 and the additional psychoactive medication(s) may be administered via the same or different routes of administration. Examples of suitable methods of administration include, but are not limited to, oral (po), intravenous (iv), intramuscular (im), subcutaneous (sc), transdermal, and rectal. Compounds may also be administered directly to the nervous system including, but not limited to, intracerebral, intraventricular, intracerebroventricular, intrathecal, intracisternal, intraspinal and / or peri-spinal routes of administration by delivery via intracranial or intravertebral needles and / or catheters with or without pump devices. The compound(s) of Formula 1 or Formula 2 and the additional psychoactive medication(s) may be administered according to simultaneous or alternating regimens, at the same or different times during the course of the therapy, concurrently in divided or single forms.

In an embodiment of the present invention is a method for the treatment of Disruptive Behavior Disorders or (DBDs) comprising administering to a subject in need thereof a combination of one or more compounds of Formula 1 or Formula 2 with one or more compounds selected from the group consisting of stimulants, including but not limited to methylphenidate, amphetamine and modafinil, major tranquilizers, such as molindone, haloperidol and chlorpromazine, minor tranquilizers, including benzodiazepines, antidepressants, including but not limited to tricyclics such as imipramine, amitriptyline, desipramine, nortriptyline, doxepin, protriptyline, trimipramine, clomipramine, amoxapine, and the like; tetracyclics such as maprotiline, and the like; non-cyclics such as nomifensine, and the like; triazolopyridines such as trazodone, and the like; serotonin reuptake inhibitors such as fluoxetine, sertraline, paroxetine, citalopram, fluvoxamine, and the like; serotonin receptor antagonists such as nefazodone, and the like; combined serotonin-noradrenergic reuptake inhibitors such as venlafaxine, milnacipran and the like; noradrenergic and specific serotonergic agents such as mirtazapine, and the like; noradrenaline reuptake inhibitors such as reboxetine, and the like; atypical antidepressants such as bupropion, and the like; natural products such as Kava-Kava, St. John's Wort, and the like; dietary supplements such as s-adenosylmethionine, and the like, mono-amine oxidase inhibitors such as phenelzine, tranylcypromine, moclobemide, and the like.

Preferably, one or more compounds of Formula 1 or Formula 2 are administered in combination with one or more compounds selected from the group consisting of: stimulants, including but not limited to methylphenidate, amphetamine and modafinil; major tranquilizers, such as molindone, haloperidol and chlorpromazine, minor tranquilizers, including benzodiazepines, antidepressants, including but not limited to tricyclics such as imipramine, amitriptyline, desipramine, nortriptyline, doxepin, protriptyline, trimipramine, clomipramine, amoxapine, tetracyclics such as maprotiline, non-cyclics such as nomifensine; triazolopyridines such as trazodone; serotonin reuptake inhibitors such as fluoxetine, sertraline, paroxetine, citalopram, fluvoxamine, serotonin receptor antagonists such as nefazodone; combined serotonin-noradrenergic reuptake inhibitors such as venlafaxine, milnacipran, noradrenergic and specific serotonergic agents such as mirtazapine, and the like; noradrenaline reuptake inhibitors such as reboxetine, atypical antidepressants such as bupropion, natural products such as Kava-Kava, St. John's Wort, dietary supplements such as s-adenosylmethionine, and the like, mono-amine oxidase inhibitors such as phenelzine, tranylcypromine, moclobemide.

More preferably, one or more compounds of Formula 1 or Formula 2 are administered in combination with one or more compounds selected from the group consisting of stimulants, major tranquilizers, mono-amino oxidase inhibitors, tricyclics and serotonin reuptake inhibitors.

Most preferably, one or more compounds of Formula 1 or Formula 2 are administered in combination with one or more compounds selected from the group consisting of stimulants and serotonin reuptake inhibitors.

As used herein, unless otherwise noted, **"halogen"** shall mean chlorine, bromine, fluorine and iodine.

As used herein, unless otherwise noted, the term **"alkyl"** whether used alone or as part of a substituent group, includes straight and branched chains. For example, alkyl radicals include methyl, ethyl, propyl, isopropyl, butyl, isobutyl, sec-butyl, t-butyl, pentyl and the like. Unless otherwise noted, **"lower"** when used with alkyl means a carbon chain composition of 1-4 carbon atoms.

As used herein, unless otherwise noted, **"alkoxy"** shall denote an oxygen ether radical of the above described straight or branched chain alkyl groups, for example, methoxy, ethoxy, n-propoxy, sec-butoxy, t-butoxy, n-hexyloxy and the like.

As used herein, the notation "*" shall denote the presence of a stereogenic center.

When a particular group is **"substituted"** (e.g., alkyl, aryl, etc.), that group may have one or more substituents, preferably from one to five substituents, more preferably from one to three substituents, most preferably from one to two substituents, independently selected from the list of substituents.

With reference to substituents, the term **"independently"** means that when more than one of such substituents is possible, such substituents may be the same or different from each other.

Under standard nomenclature used throughout this disclosure, the terminal portion of the designated side chain is described first, followed by the adjacent functionality toward the point of attachment. Thus, for example, a **"phenyl-alkyl-amino-carbonyl-alkyl"** substituent refers to a group of the formula

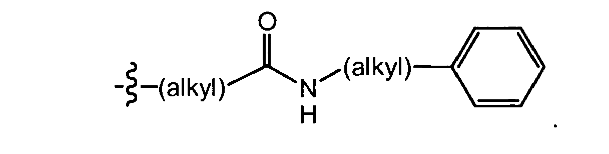

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

For use in medicine, the salts of the compounds of this invention refer to non-toxic **"pharmaceutically acceptable salts."** Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:
acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutically acceptable salts include the following:
acids including acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and
bases including; ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

Compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

The present invention further comprises pharmaceutical compositions containing one or more compounds of formula (I) with a pharmaceutically acceptable carrier. Pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this invention, one or more compounds of the present invention as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular.

In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques.

For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above.

The pharmaceutical compositions herein will contain, per unit dosage unit, e.g., tablet, capsule, powder, injection, suppository, teaspoonful and the like, of from about 0.1-1000 mg and may be given at a dosage of from about 0.01-200.0 mg/kg/day, preferably from about 0.1 to 100 mg/kg/day, more preferably from about 0.5-50 mg/kg/day, more preferably from about 1.0-25.0 mg/kg/day or any range therein. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, auto injector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection.

For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof.

When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 1000 mg of the active ingredient of the present invention.

The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinylpyrrolidone or gelatin.

The method of treating Disruptive Behavior Disorders or (DBDs) described in the present invention may also be carried out using a pharmaceutical composition comprising any of the compounds as defined herein and a pharmaceutically acceptable carrier. The pharmaceutical composition may contain between about 0.1 mg and 1000 mg, preferably about 50 to 700 mg, of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings.

Compositions suitable for oral administration include solid forms, such as pills, tablets, caplets, capsules (each including immediate release, timed release and sustained release formulations), granules, and powders, and liquid forms, such as solutions, syrups, elixirs, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methyl-cellulose and the like. For parenteral administration, sterile suspensions and solutions are desired. Isotonic preparations which generally contain suitable preservatives are employed when intravenous administration is desired.

Compounds of this invention may be administered in any of the foregoing compositions and according to dosage regimens established in the art whenever treatment of Disruptive Behavior Disorders or (DBDs) is required.

The daily dosage of the products may be varied over a wide range from 0.01 to 200 mg / kg per adult human per day. For oral administration, the compositions are preferably provided in the form of tablets containing, 25.0, 50.0, 100, 150, 200, 250, 400, 500, 600, 750 and 1000 milligrams of the active ingredient for the symptomatic adjustment of the dosage to the patient to be treated. An effective amount of the drug is ordinarily supplied at a dosage level of from about 0.1 mg/kg to about 200 mg/kg of body weight per day. Preferably, the range is from about 1.0 to about 20.0 mg/kg of body weight per day, more preferably, from about 2.0 mg/kg to about 15 mg/kg, more preferably, from about 4.0 to about 12.0 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that, both *in vivo* and *in vitro* trials using suitable, known and generally accepted cell and / or animal models are predictive of the ability of a test compound to treat or prevent a given disorder.

One skilled in the art will further recognize that human clinical trails for the indication of depression, including first-in-human, dose ranging and efficacy trials, in healthy patients and / or those suffering from a given disorder, may be completed according to methods well known in the clinical and medical arts.

### EXAMPLES

The following Examples are set forth to aid in the understanding of the invention, and are not intended and should not be construed to limit in any way the invention set forth in the claims which follow thereafter. All the examples below utilized one of the compounds of the invention. This compound is shown as Formula 7 above and will be referred to in the examples below as COMPOUND #7. This compound is also referred to as JnJ 10234094 in some of the figures, other identifiers used for this compound are RWJ 333369 and carisbamate. The structure of COMPOUND #7 is shown below;

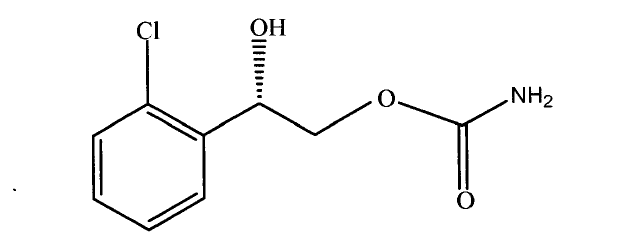

### Example 1

### Dominant-Submissive Rat In Vivo Assay

### Effects of COMPOUND #7 in the Dominant-Submissive Reaction, Animal Model of Mania and Depression (DD02313)

In this study, the effects of COMPOUND #7 on dominant or submissive behavior in pairs of rats competing for food are examined. It has been shown that anti-manic drugs, including anticonvulsants, decrease dominance and antidepressant drugs reduce submissiveness. This model uses dominant behavior as a model of mania and submissive behavior as a model of depression. The dominance and submissiveness is defined in a competition test and measured as the relative success of two food-restricted rats to gain access to a feeder. Rats are randomly paired and placed in an apparatus allowing them to compete for a food reward. The dominant-submissive relationship develops over a 2-week period. The submissive or dominant animals in pairs selected after 2 weeks of training were treated orally twice a week (b.i.d.) with COMPOUND #7 at 3 or 30 mg/kg for 5 weeks. The partner of the drug-treated animal was treated with vehicle.

A dose of 30 mg/kg of COMPOUND #7 increased competitiveness of both dominant and submissive rats. However, the effect of COMPOUND #7 on submissive rats was more extensive and had a faster onset. This effect was significant in submissive rats after the 1 st week of treatment, while for dominant rats it was significant after the 2nd week of treatment. COMPOUND #7 at 3 mg/kg produced different effects in dominant and submissive rats. It decreased competitiveness of dominant rats and did not have an effect on submissive rats. The conclusion of the study was that COMPOUND #7 may act as an antidepressant at higher doses, and at lower doses, this agent may exhibit mood-stabilizing properties in acute mania.

The objective of this study was to determine if COMPOUND #7 is active in the Reduction of Submissive Behavior Model (RSBM) of depression and the Reduction of Dominant Behavior Model (RDBM) of mania. Measurements were made at two doses (3 and 30 mg/kg) after oral twice a day (b.i.d.) administration. The effect of the drug in the RSBM was compared to the effect of fluoxetine (10 mg/kg) and vehicle (0.5% methylcellulose). The effect of the drug in the RDBM was compared with the effect of lithium (100 mg/kg) and vehicle (0.5% methylcellulose). The endpoints measured were the development of a significant reduction of submissive or dominant behavior and its time of onset.

It has been shown that dominant behavior can serve as a model of mania and submissive behavior as a model of depression. (Malatynska E, et al. Reduction of submissive behavior in rats: a test for antidepressant drug activity. Pharmacology 2002; 64:8. and Malatynska E, et al. Dominant behavior measured in a competition test as a model of mania. In: International Behavioral Neuroscience Society Meeting, ed. IBNSCapri, Italy, 2002, p 26).

Treatment of the submissive subject for 3 weeks with imipramine, desipramine, or fluoxetine significantly and dose-dependently (fluoxetine) reduced submissive behavior. The effect was attenuated after cessation of treatment with desipramine. Treatment of submissive rats with the anxiolytic diazepam, (See, Malatynska E, Goldenberg R, Shuck L, Haque A, Zamecki P, Crites G, Schindler N, Knapp RJ. Reduction of submissive behavior in rats: a test for antidepressant drug activity. Pharmacology 2002;64:8) or the psychostimulant amphetamine (unpublished observation) were ineffective.

Gardner has suggested that dominant behavior is related to mania (for a review on the relation of dominant-submissive behavior to mania and depression see Gardner, 1982). (See, Gardner R Jr. Mechanisms in manic-depressive disorder: an evolutionary model. Arch Gen Psychiatry 1982;39:1436.

We have shown that drugs commonly used to alleviate mania in the clinic such as lithium chloride, sodium valproate, carbamazepine, and clonidine significantly reduced competitive behavior when administered to dominant rats. (See, Malatynska E, Rapp R, Crites G. Dominant behavior measured in a competition test as a model of mania. In: International Behavioral Neuroscience Society Meeting, ed. IBNSCapri, Italy, 2002, p 26)

The onset of these effects for all drugs tested was similar to the onset of their therapeutic effect in patients. Thus, submissive behavior was sensitive to and selectively reduced by antidepressants. Dominant behavior was sensitive to a range of drugs used to treat mania in humans.

### FORMATION OF DOMINANT-SUBMISSIVE RELATIONSHIP (DSR)

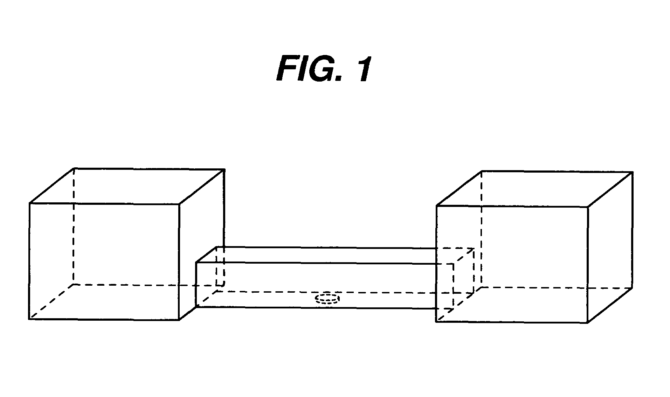

The DSR developed by two rats competing for food uses the apparatus in Figure 1. The methodology and equipment are described in several publications. (See, Malatynska E, Goldenberg R, Shuck L, Haque A, Zamecki P, Crites G, Schindler N, Knapp RJ. Reduction of submissive behavior in rats: a test for antidepressant drug activity. Pharmacology 2002;64:8; Malatynska E, Rapp R, Crites G. Dominant behavior measured in a competition test as a model of mania. In: International Behavioral Neuroscience Society Meeting, ed. IBNSCapri, Italy, 2002, p 26.; Bonnet U. Moclobemide: therapeutic use and clinical studies. CNS Drug Rev 2003;9:97; Danysz W, Plaznik A, Kostowski W, Malatynska E, Jarbe TU, Hiltunen AJ, Archer T. Comparison of desipramine, amitriptyline, zimeldine and alaproclate in six animal models used to investigate antidepressant drugs. Pharmacol Toxicol 1988;62:42; Knapp RJ, Goldenberg R, Shuck C, Cecil A, Watkins J, Miller C, Crites G, Malatynska E. Antidepressant activity of memory-enhancing drugs in the reduction of submissive behavior model. Eur J Pharmacol 2002;440:27; Kostowski W, Malatynska E, Plaznik A, Dyr W, Danysz W. Comparative studies on antidepressant action of alprazolam in different animal models. Pol J Pharmacol Pharm 1986;38,471; Malatynska E, De Leon I, Allen D, Yamamura HI. Effects of amitriptyline on GABA-stimulated 36Cl- uptake in relation to a behavioral model of depression. Brain Res Bull 1995;37:53; Malatynska E, Kostowski W. The effect of antidepressant drugs on dominance behavior in rats competing for food. Pol J Pharmacol Pharm 1984;36:531; Malatynska E, Kostowski W. Desipramine antagonizes clonidine-induced suppression of dominance in rats: possible involvement of amygdaloid nuclei. Pol J Pharmacol Pharm 1988;40:357).

In the experiments described in this report Sprague-Dawley rats weighing 160 to 180 g were used. Testing for the development of a DSR between paired rats begins with the random assignment of rats into pairs. Rats from pairs are housed separately between test sessions with other animals in groups of four. The animals are food-deprived overnight with free access to water.

The test involves placing each member of a pair in opposite chambers of the testing apparatus. These chambers are connected through a narrow tunnel with a small container of sweetened milk at the center. Only one animal at the time can have comfortable access to the feeder. The test is conducted once a day over a 5-minute period and the time spent on the feeder by each animal is recorded. At the end of the 5-minute testing period the animals are separated, returned to their home cages and given free access to food (regular small laboratory animals chow) for a limited period of time (1 hour). The testing is suspended during weekends and the animals have free access to food during this time.

During the 1st week (5 days) of testing, animals habituate to the new environment. During this 1 st week (5 days) of testing the drinking scores vary considerably and these data are used only to detect any apparent reversals within the pairs of tested rats. Dominance is assigned to the animal with the highest score during the 2nd week of testing if three criteria are achieved. First, there must be a significant difference (two-tailed t-test, P <0.05) between the average daily drinking scores of both animals. Second, the dominant animal score must be at least 40% greater than the submissive animal's score. Third, there should be no reversals during the 2-week observation process. About 25% of the initial animal pairs achieve these criteria. Only these selected pairs are continued in the study for the next 3 to 6 weeks.
Table 1 shows the time necessary and number of animals required completing one experimental unit for studying either one drug at one dose or one animal strain, to have sufficient results for valid statistical analysis. The number of animals shown in the table is typical for manual scoring.

**Table 1:**

| **Timetable for Basic Experimental Unit** | | | | |
|---|---|---|---|---|
| Procedure | Time | N of Animals | N of Animals Selected | N of Pairs with D/S Relation |
| 1 st Week (habituation) | 5 day | 32 | | |
| 2nd Week (selection) | 5 day | 32 | 10-14 | 5-7 |
| Drug | 3-6 week | 10-14 | | 5-7 |
| Administration | | | | |

| | | | | |
|---|---|---|---|---|
| D/S = dominance/submissive N = number of animals | | | | |

### DRUG TREATMENT

COMPOUND #7 was evaluated in the Rat Reduction of Submissive Behavior Model (RSBM) of depression (Malatynska, E., Rapp, R., Harrawood, D., and Tunnicliff, G., Neuroscience and Biobehavioral Review, 82 (2005) 306-313; Malatynska, E., and Knapp, R.J., Neuroscience and Biobehavioral Review, 29 (2005) 715-737).

In the experiments described in this report five submissive rats were treated b.i.d., p.o. with COMPOUND #7 at 3 mg/kg and another five submissive rats were treated with COMPOUND #7 at 30 mg/kg for 5 weeks. The dominant rats from all these pairs were treated (b.i.d., p.o.) with vehicle (0.5% methylcellulose). The data were compared to the results from our previous experimental set where submissive rats were treated intraperitoneally (i.p.) once a day with fluoxetine (10 mg/kg) and dominant rats from these pairs were treated with vehicle (water), n=6.

In a separate set of experiments five dominant rats from two sets of paired animals were treated (b.i.d., p.o.) with either 3 or 30 mg/kg COMPOUND #7 for 5 weeks. The submissive rats from these pairs were treated (b.i.d., p.o.) with vehicle (0.5% methylcellulose). The data were compared to the results from our previous experimental set where dominant rats were treated i.p. with lithium chloride (100 mg/kg) and submissive rats from these pairs were treated with vehicle (water), n=4.

There was a control group for both sets of experiments with COMPOUND #7 to show the stability of the DSR where both rats from the pair, dominant and submissive, were treated with 0.5% methylcellulose, n=8.

### DATA PROCESSING AND STATISTICAL ANALYSIS

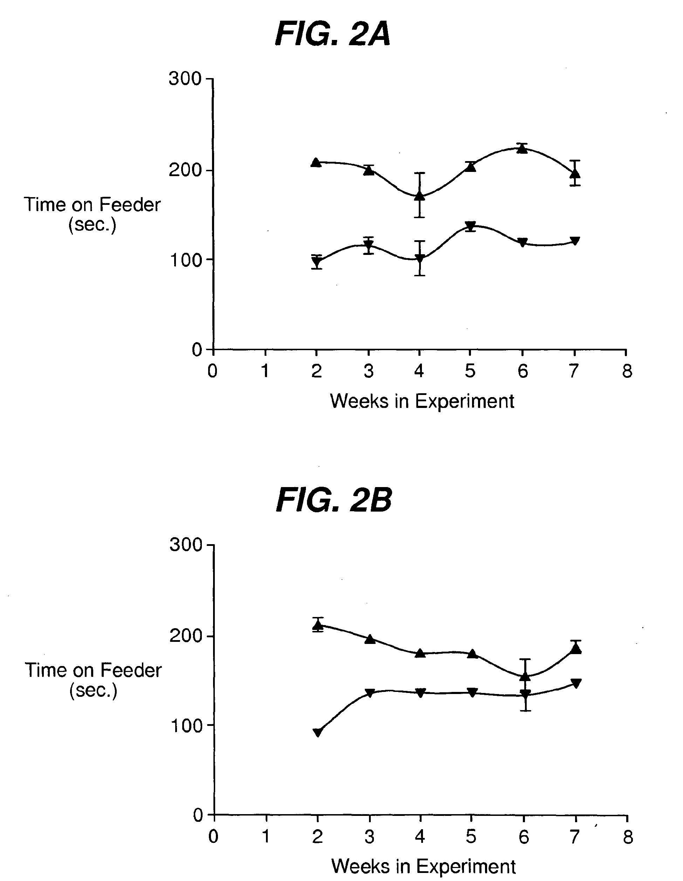

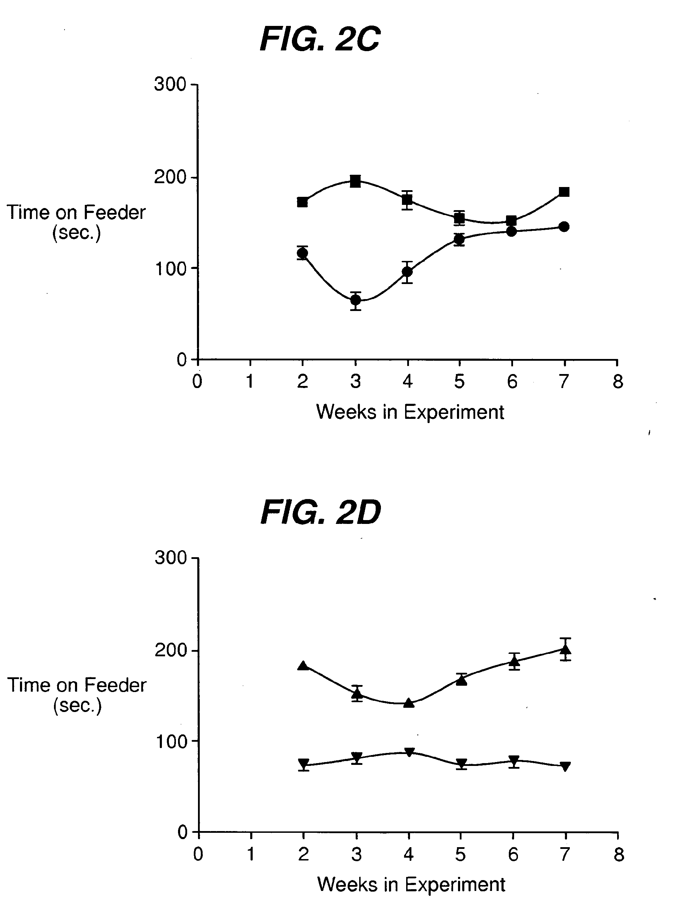

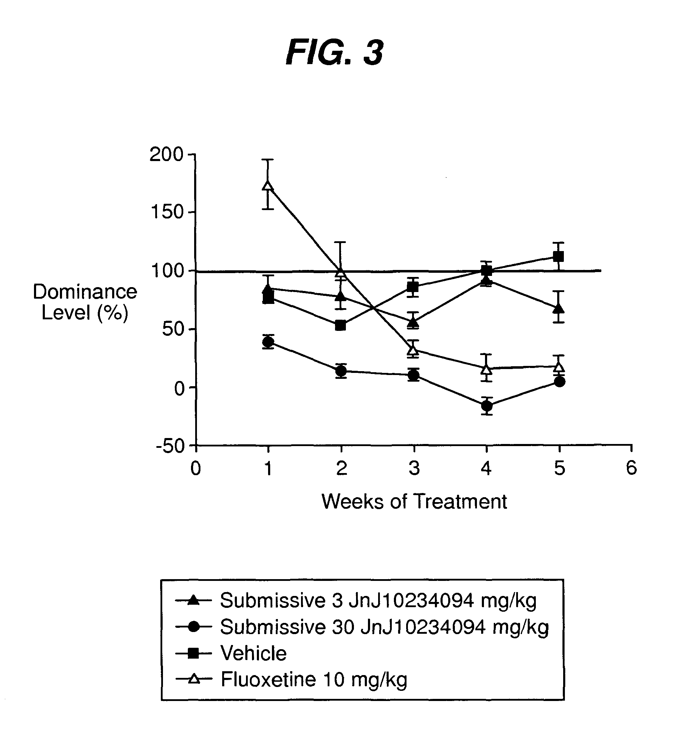

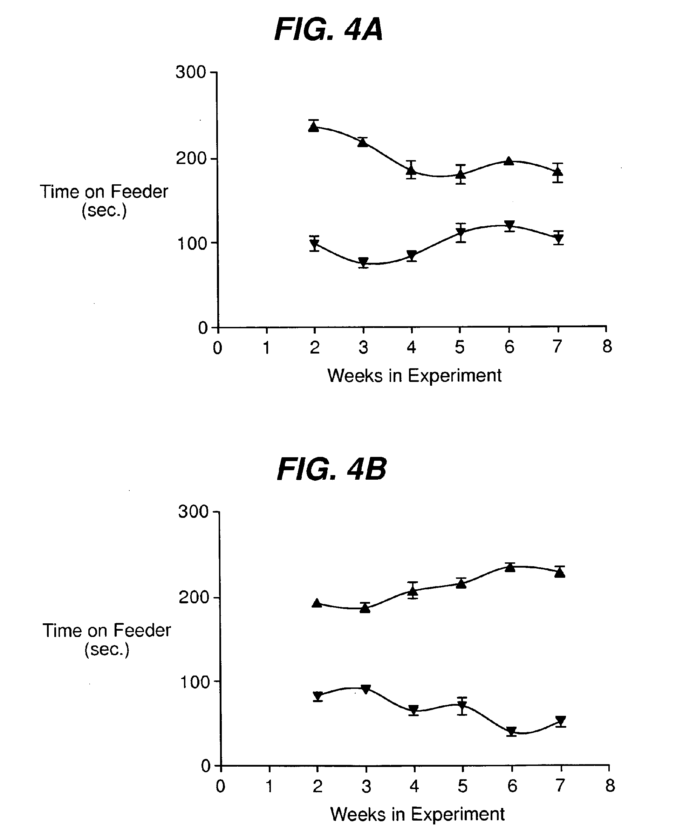

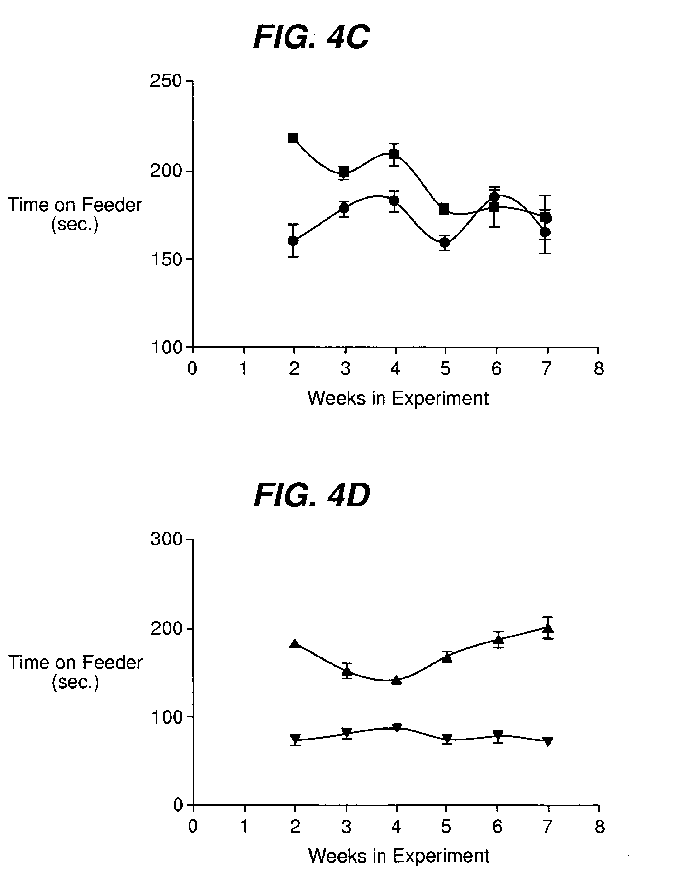

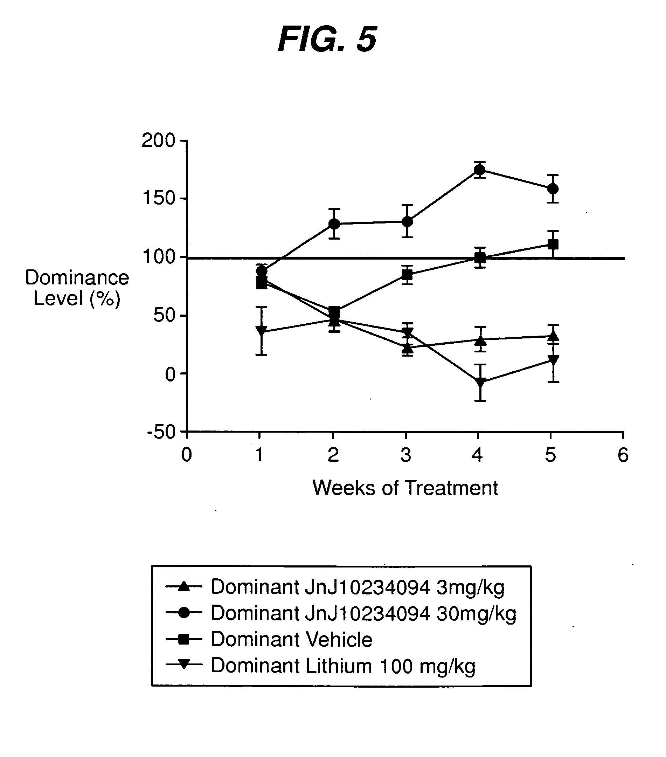

Endpoint measured in these experiments was time spent on feeder by individual rats from the pair during 5-minute daily session. Then, the average from the week was calculated (Figures 2 and 4). The treatment effect is often better captured as dominance level of the pair, because performance of the vehicle-treated paired rat is to some extent dependent on the performance of drug-treated rat. Dominance level is defined as the difference in averaged daily drinking scores for a 5-day week and reflects behavior of both animals in pair. The level of performance for different pairs of dominant and submissive rats may vary in the 2nd week of the study so the data for all rats were normalized to this initial week level (Figures 3 and 5). Thus, % of dominance level was calculated according the formula %DL = (T_{D} - T_{S}) week n x 100 / (T_{D} - T_{S}) week 2 with DL = dominance level, T_{D} = time spent by dominant rat, T_{S} = time of submissive rat, week n = test week n, week 2 (Figures 2 and 4) or 0 (Figures 3 and 5) = initial (selection) week.

The significant difference in the time spend on feeder by paired rats was calculated using two tailed t-test (Microsoft Excel). The significant differences between time spent on feeder by rats treated with different drugs were determined by analysis of variance (ANOVA) followed by Bonferroni multiple comparisons test using GraphPad Prism software (GraphPad Prism Software, Inc., San Diego, CA).

Figures 2 and 4 show data representing performance of paired dominant and submissive rats in the food competition test. In the experiment depicted in Figures 2A and 2B submissive rats and on Figures 4A and 4B dominant rats were treated with 3 or 30 mg/kg of COMPOUND #7. The respective partner rats were always treated with vehicle. The positive and negative control data are shown on panels C and D of Figures 2 and 4. The positive control for submissive rat treatment was provided by the serotonin reuptake inhibitor, fluoxetine (10 mg/kg, Figure 2C) and for dominant rat treatment with the antimanic drug, lithium (100 mg/kg, Figure 4C). Dominant and submissive rats in the pair treated simultaneously with vehicle provided negative controls for both experimental sets (Figures 2D and 4D). The dependent variable in these experiments was time spent on the feeder in seconds (y axis) and the independent variable was duration of the experiments in weeks (x axis). The habituation week data are omitted. The data plotted start on the 2nd week referred to as the initial week or selection week. In this week the performance of all dominant and submissive rats are significantly different. This significance is lost if the treatment has an effect or remains stable if the treatment does not have an effect.

It should be noted from Figures 2 and 4 that the drug mostly affects treated animal, observed as increased competitiveness of submissive rat treated with antidepressant or decreased competitiveness of dominant rat treated with antimanic drug. The transformed data as described in the Methods section (3.3) are presented in Figures 3 and 5. The dominance level of the initial week is marked as 100% for Week 0, before treatment week. The values of dominance level in the following after treatment weeks, 1-5, (x axis) are presented as data transformed according to the above discussed formula, (Methods section, 3.3). The data are presented on Figure 3 for treatment of submissive rat from the pair and on Figure 5 for treatment of dominant rat from the pair. This comparison confirms the effects observed in raw data and facilitates comparison of treatment effects.

### EFFECTS OF COMPOUND #7 ON SUBMISSIVE RATS

COMPOUND #7 at 3 mg/kg did not have any effect on submissive rat behavior, similar to vehicle-treated submissive rats (Figures 2A and 2D). However, at the higher dose (30 mg/kg), COMPOUND #7 significantly increased competitiveness of submissive rat (Figures 2B and 3) compared to vehicle-treated submissive rats on the level of the corresponding week (Figure 2D and 3). This was similar to fluoxetine-treated submissive rats. COMPOUND #7 (Figures 2C and 3).

Thus, COMPOUND #7 has the same efficacy as fluoxetine but the onset of this effect was faster. The COMPOUND #7 (30 mg/kg) increased competitiveness of submissive rats after 1 week of treatment while the fluoxetine effect was only significant after 3 weeks of treatment.

### EFFECTS OF COMPOUND #7 ON DOMINANT RATS

COMPOUND #7 at 3 mg/kg decreased the performance of dominant rats (Figures 4A and 5). This effect was significant after 3 weeks of treatment. The extent and onset of the effect was not significantly different than the effect of lithium (Figures 4C and 5). At the higher dose (30 mg/kg) COMPOUND #7 significantly increased the competitiveness of dominant rats (Figure 4B) as compared to water treated dominant rats (Figures 4D and 5). This effect was opposite the effect of lithium and COMPOUND #7's effect at the 3 mg/kg dose level. The onset of this effect occurred after 2 weeks of treatment.

The major finding of this study is that COMPOUND #7 affects the competitive behavior of both dominant and submissive rats. Effects of COMPOUND #7 to decrease dominant behavior and to increase competitiveness of submissive rats occurred at different doses. While dominant behavior was reduced at 3-mg/kg dose, the reduction of submissive behavior was most pronounced at 30 mg/kg. The 30-mg/kg dose increased competitiveness of both dominant and submissive rats. However, the effect of COMPOUND #7 on submissive rats was more extensive and with a faster onset. This effect was significant in submissive rats after the 1 st week of treatment, while for dominant rats it was significant only after the 4th week of treatment. Because dominant behavior of competing rats was shown to model mania and submissive behavior was shown to model depression (See, Malatynska E, Goldenberg R, Shuck L, Haque A, Zamecki P, Crites G, Schindler N, Knapp RJ. Reduction of submissive behavior in rats: a test for antidepressant drug activity. Pharmacology 2002;64:8; Malatynska E, Rapp R, Crites G. Dominant behavior measured in a competition test as a model of mania. In: International Behavioral Neuroscience Society Meeting, ed. IBNSCapri, Italy, 2002, p 26) it is possible that COMPOUND #7 may have mood stabilizing activity in both phases of bipolar disorders, depression, and mania.

Dominant-submissive behavior between animals can model human mood disorders. Submissive behavior has features of human depression that can be modeled using rats or mice in a behavioral paradigm referred to as the RSBM in which submissive behavior is reduced by antidepressant drugs. An analogous approach referred to as RDBM is sensitive to drugs used to treat mania. Neither model, RDBM or RSBM, is a complete model of bipolar disorder but they can be used together to model individual poles of bipolar symptoms. At this time the RSBM is better established than the RDBM. The studies confirming the validity of RDBM model should be extended. This study shows clearly that rats with different behavioral traits react differently to the same anticonvulsant agent. This is an important finding since diverse response to treatment occurs also in the clinic. Only about 40 to 70% of manic or depressive patients respond to a given antimanic or antidepressant drug, and the reason for this limitation is not known. Further work with this model could shed light on the mechanisms of the resistance to treatment.

We conclude that COMPOUND #7 dose dependently increases competitiveness of submissive rats therefore may act as an antidepressant. COMPOUND #7 at lower dose reduces dominant rat behavior. Thus, this agent may exhibit mood-stabilizing properties in acute mania at lower dose.

### REFERENCES

1. Malatynska E, Goldenberg R, Shuck L, Haque A, Zamecki P, Crites G, Schindler N, Knapp RJ. Reduction of submissive behavior in rats: a test for antidepressant drug activity. Pharmacology 2002;64:8.
2. Malatynska E, Rapp R, Crites G. Dominant behavior measured in a competition test as a model of mania. In: International Behavioral Neuroscience Society Meeting, ed. IBNSCapri, Italy, 2002, p 26.
3. Gardner R Jr. Mechanisms in manic-depressive disorder: an evolutionary model. Arch Gen Psychiatry 1982;39:1436.
4. Ernst CL, Goldberg JF. Antidepressant properties of anticonvulsant drugs for bipolar disorder. J Clin Psychopharmacol 2003;23:182.
5. Carpenter LL, Leon Z, Yasmin S, Price LH. Do obese depressed patients respond to topiramate? A retrospective chart review. J Affect Disord 2002;69:251.
6. McElroy SL, Zarate CA, Cookson J, Suppes T, Huffman RF, Greene P, Ascher J. A 52-week, open-label continuation study of lamotrigine in the treatment of bipolar depression. J Clin Psychiatry 2004;65:204.
7. Bonnet U. Moclobemide: therapeutic use and clinical studies. CNS Drug Rev 2003;9:97.
8. Danysz W, Plaznik A, Kostowski W, Malatynska E, Jarbe TU, Hiltunen AJ, Archer T. Comparison of desipramine, amitriptyline, zimeldine and alaproclate in six animal models used to investigate antidepressant drugs. Pharmacol Toxicol 1988;62:42.
9. Knapp RJ, Goldenberg R, Shuck C, Cecil A, Watkins J, Miller C, Crites G, Malatynska E. Antidepressant activity of memory-enhancing drugs in the reduction of submissive behavior model. Eur J Pharmacol 2002;440:27.
10. Kostowski W, Malatynska E, Plaznik A, Dyr W, Danysz W. Comparative studies on antidepressant action of alprazolam in different animal models. Pol J Pharmacol Pharm 1986;38,471.
11.Malatynska E, De Leon I, Allen D, Yamamura HI. Effects of amitriptyline on GABA-stimulated 36Cl- uptake in relation to a behavioral model of depression. Brain Res Bull 1995;37:53.
12. Malatynska. E, Kostowski W. The effect of antidepressant drugs on dominance behavior in rats competing for food. Pol J Pharmacol Pharm 1984;36:531.
13. Malatynska E, Kostowski W. Desipramine antagonizes clonidine-induced suppression of dominance in rats: possible involvement of amygdaloid nuclei. Pol J Pharmacol Pharm 1988;40:357.

### Example 2

### Effects of COMPOUND #7 in the Isolation Induced Aggression Model

The test compound used in this report is the same as that used in Example 1 and referred to therein as COMPOUND #7 shown as Formula #7 in the specification of this patent application. As discussed in detail below, data from the experiment showed that the test compound administered at doses of 40-mg/kg p.o. inhibited isolation-induced aggressive behavior in pairs of mice tested one hour after administration. This anti-aggressive effect of the test compound was not related to sedation.

The pharmacological effect shown by the test compound in inhibiting isolation induced aggression in this animal model suggests that this compound would have a beneficial effect on aggressiveness in humans and could improve impulse control and therefore is likely to be useful as a treatment for Disruptive Behavioral Disorders (DBDs) in humans

The effects of test compound on the aggressive behavior of Crl:CD-1(ICR)BR albino mice were evaluated using the paradigm of isolation-induced aggression.

Test compound at 40-mg/kg p.o. statistically significantly inhibited isolation-induced aggressive behavior in pairs of mice tested 1 hour after administration (p <0.05). Test compound at 20-mg/kg p.o. also significantly shortened the onset of initiation of fighting in the treated group compared to the corresponding vehicle-treated group 4 hours after administration. The effects of test compound on general behavior were evaluated by visual observation using a checklist of behaviors. No behavioral or physical signs were observed in mice administered test compound at doses up to 100 mg/kg p.o., the test compound, administered at 300 mg/kg p.o., produced sedation.. Results suggest that the anti-aggressive activity of test compound at 40 mg/kg p.o. is not related to sedation.

Male Crl:CD-1 (ICR)BR albino mice, fasted overnight, were individually housed for 5 weeks in plastic cages on wood-chip bedding. Subsequently, they were paired for 1 minute daily for several days by placing one individually housed mouse (intruder) into the resident cage of another individually housed mouse (resident). The 1-minute pairing of intruder and resident mouse elicits aggressive behavior. Pairs of mice showing consistent aggressive behavior when paired for 1 minute, during several days were selected as subjects for drug testing.

Test compound (10 to 40 mg/kg p.o.) or the vehicle (methocel; aqueous 0.5%, w/v, hydroxypropyl methylcellulose solution) was administered (10 mL/kg) to the resident and intruder mouse. One and 4 hours after dosing, the mice were paired and the onset of fighting was recorded. Pairs of mice that did not fight within 1 minute were separated. The duration of fighting during a 1-minute test period was recorded. Mice were re-used in this procedure after several days to a week to allow metabolism and elimination of test compounds. The mice weighed 32 to 49 g at the time of testing and were fasted overnight before dosing. Mice were euthanized with CO2 if they were sick or injured.

Results were expressed as the median onset of fighting and median duration of fighting in vehicle and drug-treated groups. The statistical significance of an increase in the median onset or a reduction in the median duration of fighting in pairs of mice given test compound or its vehicle 1 and 4 hours after administration was determined using the nonparametric Wilcoxon test (p <0.05, 1-tailed).

Test compound at 40-mg/kg p.o. inhibited isolation-induced aggressive behavior in pairs of mice tested 1 hour after administration, as shown by a statistically significant reduction (p <0.05; Wilcoxon rank sums, 1-tailed) in the median duration of fighting compared to that in the corresponding vehicle-treated group (See Tables 1A) When testing was repeated 4 hours after administration, the onset for initiation of fighting in the group that was administered test compound at 20 mg/kg p.o. was statistically significantly shorter than that in the corresponding vehicle-treated group. (See Table 1 B)

The biological significance of this reduction in onset of fighting is unclear, because the median duration of fighting was not affected in any group that was administered test compound and tested 4 hours after administration compared to the corresponding vehicle-treated groups (See Tables 1A and 1 B below).

In conclusion, the anti-aggressive activity of test compound at 40-mg/kg p.o. was not related to sedation, because no CNS-related effects were observed in other mice up to 4 hours after administration of test compound at 40 or 100-mg/kg p.o. although test compound at 300-mg/kg p.o. produced CNS-related effects in mice under the conditions of testing used.(See Table 2)

**TABLE 1 A**

| EFFECT OF TEST COMPOUND ON MEDIAN ONSET AND DURATION OF ISOLATION-INDUCED FIGHTING IN PAIRS OF MICE | | | | | | |
|---|---|---|---|---|---|---|
| **1 HOUR AFTER ADMINISRATION** | | | | | | |
| Treatment | mg/kg p.o. | N^{a} | Median Onset (sec) | P | Median Duration (sec) | P |
| Vehicle | 0 | 11 | 13 | --- | 19 | --- |
| Test Compound | 10 | 12 | 5 | 0.174 | 28 | 0.289 |
| Vehicle | 0 | 10 | 8 | --- | 14 | --- |
| Test Compound | 20 | 10 | 11 | 0.259 | 20 | 0.325 |
| Vehicle | 0 | 18 | 13 | --- | 19 | --- |
| Test Compound | 30 | 17 | 14 | 0.375 | 18 | 0.5 |
| Vehicle | 0 | 10 | 8 | --- | 14 | --- |
| Test Compound | 40 | 10 | 40 | 0.071 | 0.5^{b} | 0.035 |

| | | | | | | |
|---|---|---|---|---|---|---|
| ^{a} Number of pairs of mice. ^{b} Compared to the vehicles treatment, run on the same day, the experimental compound produced either a statistically significant increase or decrease in onset or duration of fighting in paired mouse-isolates (p<0.05; Willcoxon rank sums, 1-tailed). | | | | | | |

**TABLE 1B**

| EFFECT OF TEST COMPOUND ON MEDIAN ONSET AND DURATION OF ISOLATION-INDUCED FIGHTING IN PAIRS OF MICE | | | | | | |
|---|---|---|---|---|---|---|
| **4 HOURS AFTER ADMINISRATION** | | | | | | |
| Treatment | mg/kg p.o. | N^{a} | Median Onset (sec) | P | Median Duration (sec) | P |
| Vehicle | 0 | 11 | 34 | --- | 2 | --- |
| Test Compound | 10 | 12 | 4 | 0.092 | 6 | 0.366 |
| Vehicle | 0 | 10 | 50 | --- | 12 | --- |
| Test Compound | 20 | 10 | 4^{b} | 0.036 | 26 | 0.078 |
| Vehicle | 0 | 18 | 34 | --- | 2 | --- |
| Test Compound | 30 | 17 | 4 | 0.32 | 9.5 | 0.215 |
| Vehicle | 0 | 10 | 50 | --- | 12 | --- |
| Test Compound | 40 | 10 | 17 | 0.255 | 20 | 0.281 |

| | | | | | | |
|---|---|---|---|---|---|---|
| ^{a} Number of pairs of mice. ^{b} Compared to the vehicles treatment, run on the same day, the experimental compound produced either a statistically significant increase or decrease in onset or duration of fighting in paired mouse-isolates (p<0.05; Willcoxon rank sums, 1-tailed). | | | | | | |

**TABLE 2**

| EFFECTS OF ORAL ADMINISTRATION OF TEST COMPOUND ON THE GENERAL BEHAVIOR OF MALE MICE | | | | | | |
|---|---|---|---|---|---|---|
| | Number of Mice Affected (N=3/group) Mg/kg p.o.^{a} | | | | | |
| | 40 | | 100 | | 300 | |
| Physical and Behavioral Signs | 1 h | 4h | 1 h | 4h | 1 h | 4h |
| Decreased Open Field Activity | 0 | 0 | 0 | 0 | 3 | 2 |
| Impaired Horizontal Screen | 0 | 0 | 0 | 0 | 3 | 2 |
| Performance | | | | | | |
| Locomotor Ataxia | 0 | 0 | 0 | 0 | 0 | 2 |
| Soft Body Tone | 0 | 0 | 0 | 0 | 3 | 2 |
| Loss of Righting Reflex | 0 | 0 | 0 | 0 | 3 | 0 |
| Loss of Corneal Reflex | 0 | 0 | 0 | 0 | 3 | 0 |
| Loss of Pinnal Reflex | 0 | 0 | 0 | 0 | 3 | 0 |
| Extensor Limb Tone (hind limb) | 0 | 0 | 0 | 0 | 3 | 2 |
| Impaired Visual Placing Reflex | 0 | 0 | 0 | 0 | 3 | 0 |
| Decreased Startle Reflex | 0 | 0 | 0 | 0 | 3 | 0 |
| Reduced Skin Plasticity | 0 | 0 | 0 | 0 | 3 | 0 |
| Tail Pinch Response, Absent | 0 | 0 | 0 | 0 | 3 | 0 |
| Passive Response to Handling | 0 | 0 | 0 | 0 | 3 | 0 |

| | | | | | | |
|---|---|---|---|---|---|---|
| ^{a} Test compound was suspended in the vehicle which consisted of 0.5%, w/v, aqueous hydroxypropyl methylcellulose, approximately 4000 cps; GFI-90000-000-E-004X, Lot 9428N. | | | | | | |

## Claims

1. A compound of Formula 1 or Formula 2: 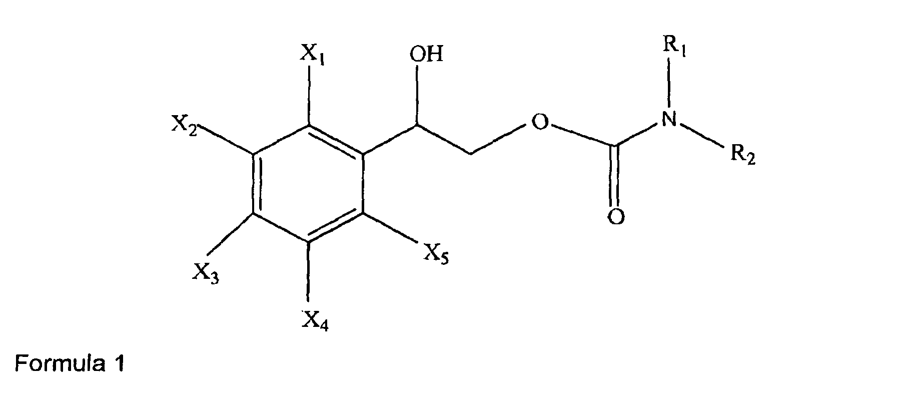 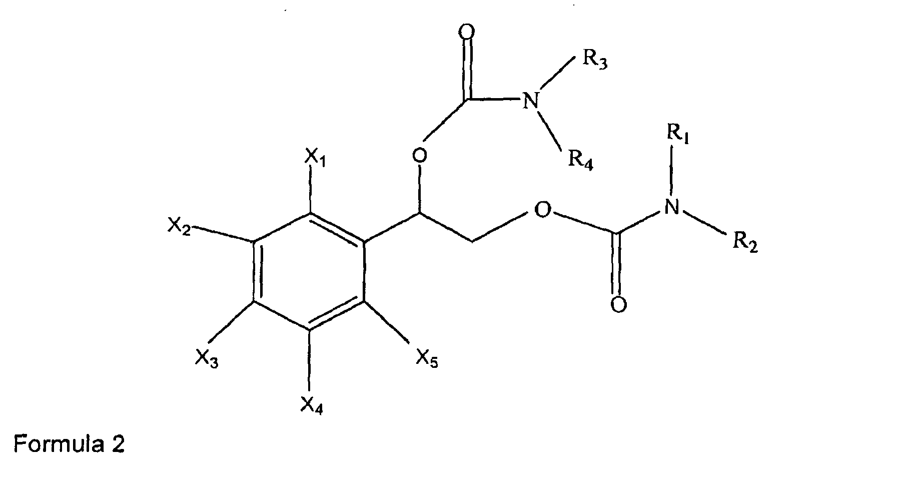 or a pharmaceutically acceptable salt or ester form thereof for use in treating Disruptive Behavior Disorders or (DBDs)
wherein:
R_{1,} R₂, R₃ and R₄ are independently hydrogen or C₁-C₄ alkyl,
wherein
C₁₋C₄ alkyl is substituted or unsubstituted with phenyl, and
wherein
phenyl is substituted or unsubstituted with up to five substituents independently selected from; halogen, C₁₋C₄ alkyl, C₁₋C₄ alkoxy, nitro,
cyano and amino
wherein amino is optionally mono or disubstituted with C₁₋C₄ alkyl,
and X₁, X₂, X₃, X₄ and X₅ are independently hydrogen, fluorine, chlorine,
bromine or iodine.

2. An enantiomer, or a pharmaceutically acceptable salt or ester thereof of a compound of claim 1, selected from the group consisting of Formula (I) and Formula (II) or enantiomeric mixture wherein one enantiomer selected from the group consisting of Formula (I) and Formula (II) predominates for use according to claim 1: 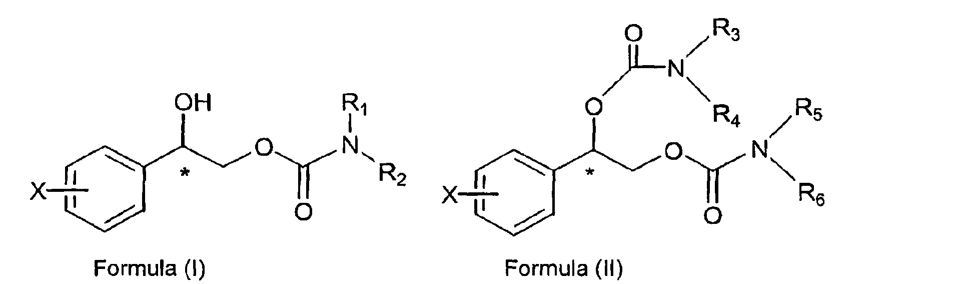 wherein
phenyl is substituted at X with one to five halogen atoms selected from the group consisting of fluorine, chlorine, bromine and iodine; and,
R₁, R₂, R₃, R₄, R₅ and R₆ are independently selected from the group consisting of hydrogen and C₁-C₄ alkyl; wherein C₁-C₄ alkyl is optionally substituted with phenyl (wherein phenyl is optionally substituted with substituents independently selected from the group consisting of halogen, C₁-C₄ alkyl, C₁-C₄ alkoxy, amino, nitro and cyano).

3. The enantiomer or enantiomeric mixture of claim 2 wherein the enantiomer selected from the group consisting of Formula (I) and Formula (II) is an enantiomer selected from the group consisting of Formula (Ia) and Formula (IIa): 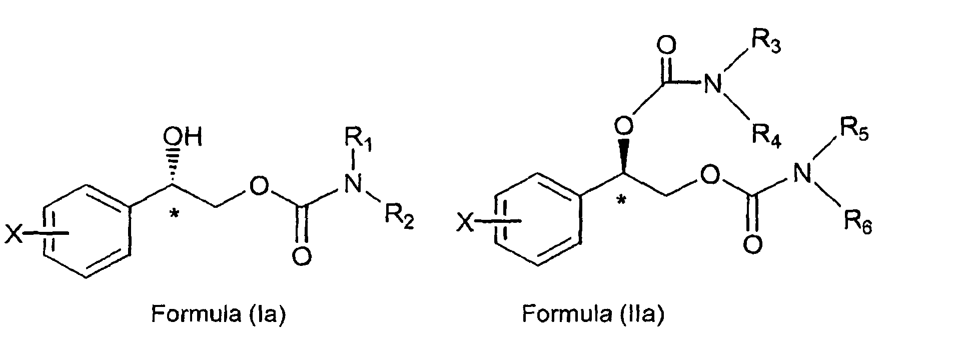 wherein
phenyl is substituted at X with one to five halogen atoms selected from the group consisting of fluorine, chlorine, bromine and iodine; and,
R₁, R₂, R₃, R₄, R₅ and R₆ are independently selected from the group consisting
of hydrogen and C₁-C₄ alkyl; wherein C₁-C₄ alkyl is optionally substituted with phenyl wherein phenyl is optionally substituted with substituents independently selected from the group consisting of halogen, C₁-C₄ alkyl, C₁-C₄ alkoxy, amino, nitro and cyano.

4. The compound, enantiomer or enantiomeric mixture of any one of claims 1, 2 or 3 wherein X is chlorine substituted at the ortho position of the phenyl ring and wherein R₁, R₂, R₃, R₄, R₅ and R₆ are selected from hydrogen.

5. The enantiomer or enantiomeric mixture of claim 2 wherein the enantiomer selected from the group consisting of Formula (I) and Formula (II) is an enantiomer selected from the group consisting of Formula (Ib) and Formula (IIb) or a pharmaceutically acceptable salt or ester form thereof:

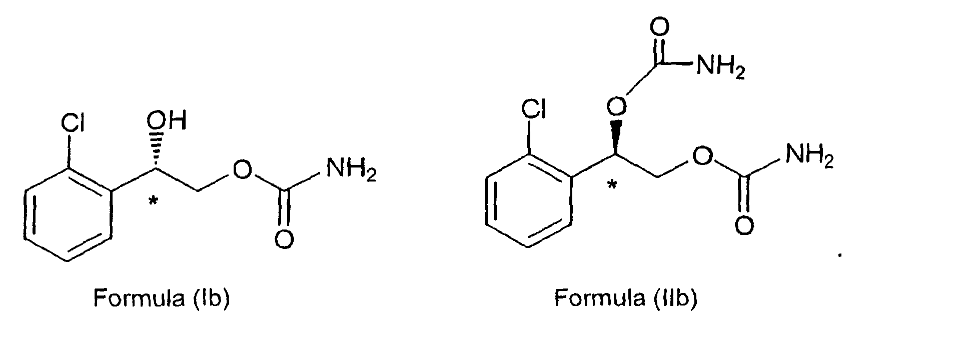

6. The enantiomer or enantiomeric mixture of any one of claims 2, 3 and 5 wherein one enantiomer selected from the group consisting of Formula (I) and Formula (II), Formula (Ia) and Formula (IIa), and Formula (Ib) and Formula (IIb) predominates to the extent of about 90% or greater.

7. The enantiomer or enantiomeric mixture of any one of claims 2, 3 and 5 wherein one enantiomer selected from the group consisting of either Formula (I) and Formula (II) or Formula (Ia) and Formula (IIa) Formula (Ib) and Formula (IIb) predominates to the extent of about 98% or greater.

8. The enantiomer or enantiomeric mixture of claim 5 wherein the enantiomer is Formula (Ib) and predominates to the extent of 98% or greater.

9. The enantiomer or enantiomeric mixture of claim 5 wherein the enantiomer is Formula (IIb) and predominates to the extent of 98% or greater.

10. An enantiomer of the compound of claim 1 of formula (Ib) which predominates to the extent of about 98% or greater for use according to claim 1:

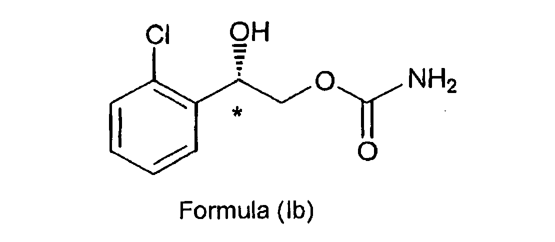

11. The compound of claim 1 or the enantiomer of claim 10, wherein the Disruptive Behavior Disorder is Conduct Disorder.

12. The compound of claim 1 or the enantiomer of claim 10, wherein the Disruptive Behavior Disorder is Oppositional Defiant Disorder.

13. The compound of claim 1, wherein the Disruptive Behavior Disorder is Disruptive Behavior Disorder Not Otherwise Specified.

14. An enantiomer of the compound of claim 1 selected from the group consisting of Formula (Ib) and Formula (IIb) or a pharmaceutically acceptable salt or ester form thereof: 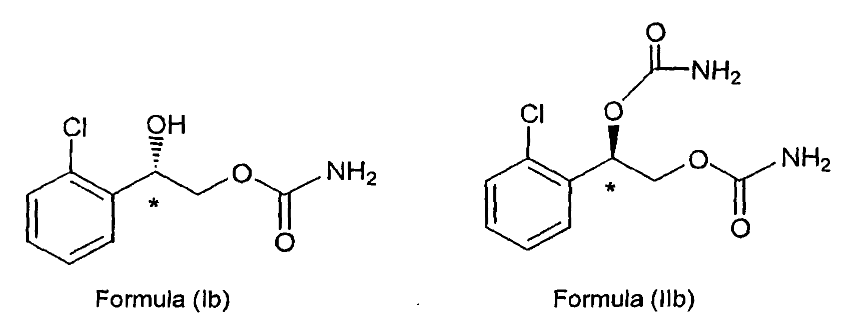 and at least one additional psychoactive medication for use in the co-therapy treatment of Disruptive Behavior Disorders.

15. The enantiomer of claims 14, wherein the compound is an enantiomer Formula Ib which predominates to the extent of about 98% or greater.

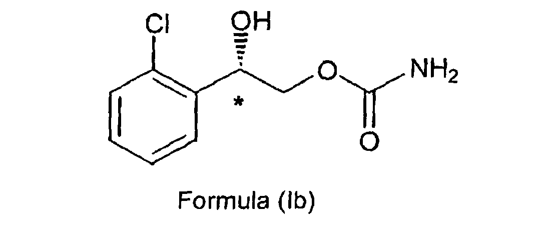

16. The enantiomer of Claim 14 wherein the additional psychoactive medication is selected from the group consisting of: stimulants, including methylphenidate, amphetamine and modafinil, major tranquilizers, such as molindone, haloperidol and chlorpromazine, minor tranquilizers, including benzodiazepines, antidepressants, including but not limited to tricyclics such as imipramine, amitriptyline, desipramine, nortriptyline, doxepin, protriptyline, trimipramine, clomipramine, amoxapine, and the like; tetracyclics such as maprotiline, and the like; non-cyclics such as nomifensine, and the like; triazolopyridines such as trazodone, and the like; serotonin reuptake inhibitors such as fluoxetine, sertraline, paroxetine, citalopram, fluvoxamine, and the like;
serotonin receptor antagonists such as nefazodone, and the like; combined serotonin-noradrenergic reuptake inhibitors such as venlafaxine, milnacipran and the like; noradrenergic and specific serotonergic agents such as mirtazapine, and the like; noradrenaline reuptake inhibitors such as reboxetine, and the like; atypical antidepressants such as bupropion, and the like; natural products such as Kava-Kava, St. John's Wort, and the like; dietary supplements such as s-adenosylmethionine, and the like, mono-amine oxidase inhibitors such as phenelzine, tranylcypromine, moclobemide.

17. The enantiomer of Claim 14, wherein the additional psychoactive medication is selected from the group consisting of: methylphenidate, amphetamine and modafinil, molindone, haloperidol and chlorpromazine, fluoxetine, sertraline, paroxetine, citalopram, fluvoxamine, nefazodone, venlafaxine, milnacipran, mirtazapine, bupropion.

## Patentansprüche

1. Eine Verbindung von Formel 1 oder Formel 2: 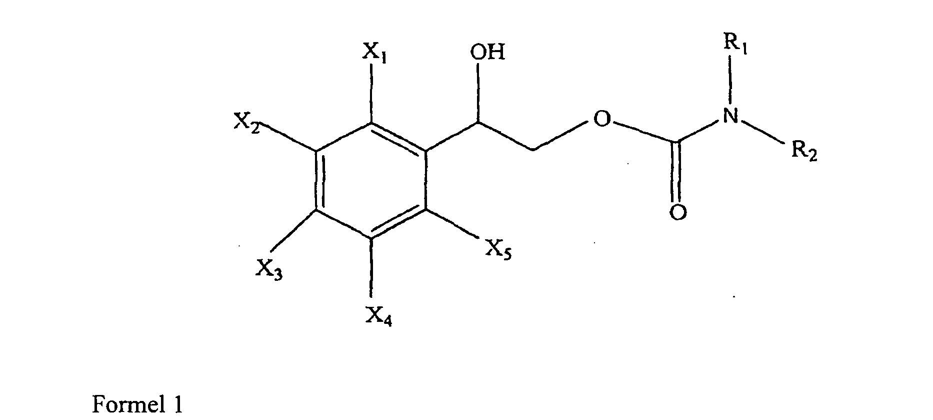 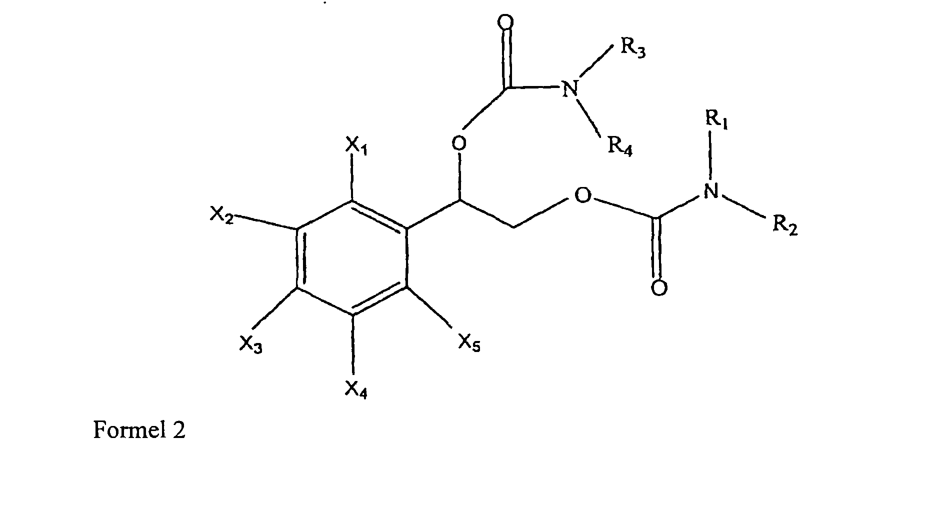 oder eine pharmazeutisch verträgliche Salz- oder Esterform davon zur Verwendung bei der Behandlung disruptiver Verhaltensstörungen oder (DBDs),
wobei:
R₁, R₂, R₃ und R₄ unabhängig Wasserstoff oder C₁-C₄-Alkyl sind,
wobei
C₁-C₄-Alkyl mit Phenyl substituiert oder unsubstituiert ist und wobei
Phenyl mit bis zu fünf Substituenten substituiert oder unsubstituiert ist, die unabhängig ausgewählt sind aus Halogen, C₁-C₄-Alkyl, C₁-C₄-Alkoxy, Nitro, Cyano und Amino,
wobei Amino gegebenenfalls einfach oder zweifach mit C₁-C₄-Alkyl substituiert ist,
und X₁, X₂, X₃, X₄ und X₅ unabhängig Wasserstoff, Fluor, Chlor, Brom oder Iod sind.

2. Ein Enantiomer, oder ein pharmazeutisch verträgliches/r Salz oder Ester davon, einer Verbindung von Formel 1, ausgewählt aus der Gruppe, bestehend aus Formel (I) und Formel (II), oder eine Enantiomerenmischung, in der ein Enantiomer, das ausgewählt ist aus der Gruppe, bestehend aus Formel (I) und Formel (II), überwiegt, zur Verwendung nach Anspruch 1: 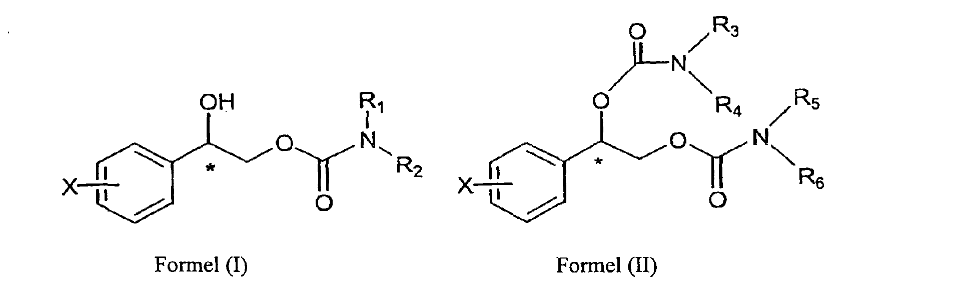 wobei
Phenyl an X mit einem bis fünf Halogenatomen substituiert ist, die ausgewählt sind aus der Gruppe, bestehend aus Fluor, Chlor, Brom und Iod; und
R₁, R₂, R₃, R₄, R₅ und R₆ unabhängig ausgewählt sind aus der Gruppe, bestehend aus Wasserstoff und C₁-C₄-Alkyl; wobei C₁-C₄-Alkyl gegebenenfalls mit Phenyl substituiert ist (wobei Phenyl gegebenenfalls mit Substituenten substituiert ist, die unabhängig ausgewählt sind aus der Gruppe, bestehend aus Halogen, C₁-C₄-Alkyl, C₁-C₄-Alkoxy, Amino, Nitro und Cyano).

3. Das Enantiomer oder die Enantiomerenmischung nach Anspruch 2, wobei das Enantiomer, das ausgewählt ist aus der Gruppe, bestehend aus Formel (I) und Formel (II), ein Enantiomer ist, das ausgewählt ist aus der Gruppe, bestehend aus Formel (Ia) und Formel (IIa): 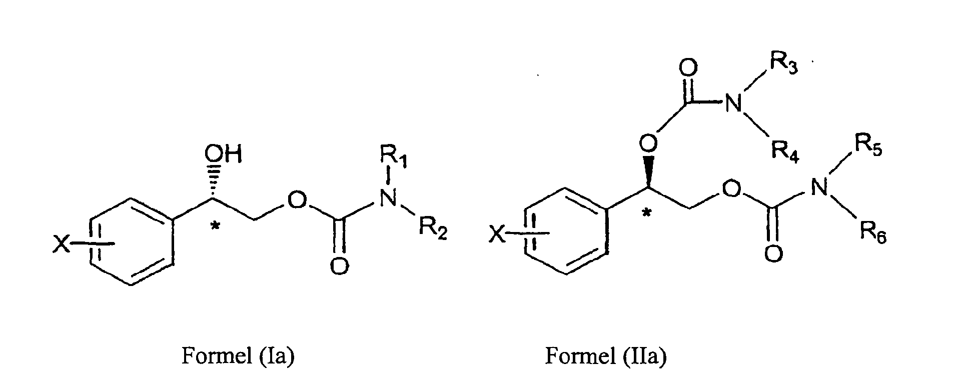 wobei
Phenyl an X mit einem bis fünf Halogenatomen substituiert ist, die ausgewählt sind aus der Gruppe, bestehend aus Fluor, Chlor, Brom und Iod; und
R₁, R₂, R₃, R₄, R₅ und R₆ unabhängig ausgewählt sind aus der Gruppe, bestehend aus Wasserstoff und C₁-C₄-Alkyl; wobei C₁-C₄-Alkyl gegebenenfalls mit Phenyl substituiert ist, wobei Phenyl gegebenenfalls mit Substituenten substituiert ist, die unabhängig ausgewählt sind aus der Gruppe, bestehend aus Halogen, C₁-C₄-Alkyl, C₁-C₄-Alkoxy, Amino, Nitro und Cyano.

4. Die Verbindung, das Enantiomer oder die Enantiomerenmischung nach einem der Ansprüche 1, 2 oder 3, wobei X Chlor ist, substituiert an der ortho-Position des Phenylringes, und wobei R₁, R₂, R₃, R₄, R₅ und R₆ ausgewählt sind aus Wasserstoff.

5. Das Enantiomer oder die Enantiomerenmischung nach Anspruch 2, wobei das Enantiomer, das ausgewählt ist aus der Gruppe, bestehend aus Formel (I) und Formel (II), ein Enantiomer ist, das ausgewählt ist aus der Gruppe, bestehend aus Formel (Ib) und Formel (IIb) oder einer pharmazeutisch verträglichen Salz- oder Esterform davon:

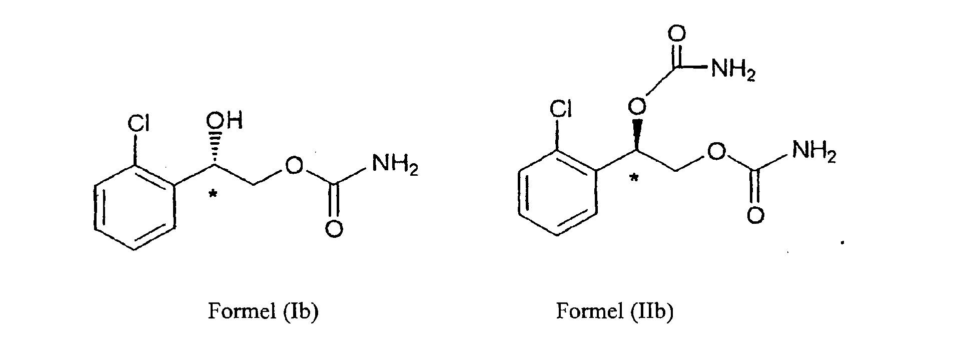

6. Das Enantiomer oder die Enantiomerenmischung nach einem der Ansprüche 2, 3 und 5, wobei ein Enantiomer, das ausgewählt ist aus der Gruppe, bestehend aus Formel (I) und Formel (II), Formel (Ia) und Formel (IIa) und Formel (Ib) und Formel (IIb), bis zum Umfang von etwa 90% oder mehr überwiegt.

7. Das Enantiomer oder die Enantiomerenmischung nach einem der Ansprüche 2, 3 und 5, wobei ein Enantiomer, das ausgewählt ist aus der Gruppe, bestehend aus entweder Formel (I) und Formel (II) oder Formel (Ia) und Formel (IIa) oder Formel (Ib) und Formel (IIb), bis zum Umfang von etwa 98% oder mehr überwiegt.

8. Das Enantiomer oder die Enantiomerenmischung nach Anspruch 5, wobei das Enantiomer Formel (Ib) ist und bis zum Umfang von 98% oder mehr überwiegt.

9. Das Enantiomer oder die Enantiomerenmischung nach Anspruch 5, wobei das Enantiomer Formel (IIb) ist und bis zum Umfang von 98% oder mehr überwiegt.

10. Ein Enantiomer der Verbindung von Anspruch 1 von Formel (Ib), das bis zum Umfang von etwa 98% oder mehr überwiegt, zur Verwendung nach Anspruch 1:

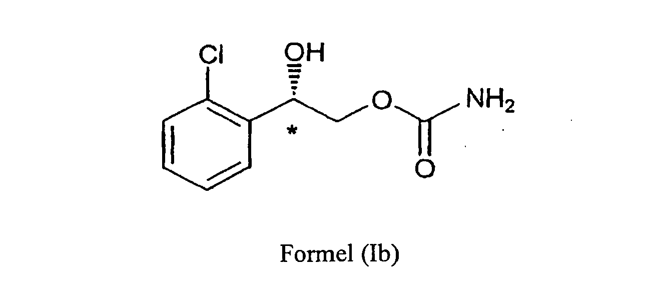

11. Die Verbindung nach Anspruch 1 oder das Enantiomer nach Anspruch 10, wobei die disruptive Verhaltensstörung Störung des Sozialverhaltens ist.

12. Die Verbindung nach Anspruch 1 oder das Enantiomer nach Anspruch 10, wobei die disruptive Verhaltensstörung Trotzverhalten ist.

13. Die Verbindung nach Anspruch 1, wobei die disruptive Verhaltensstörung nicht sonst spezifizierte disruptive Verhaltensstörung ist.

14. Ein Enantiomer der Verbindung von Anspruch 1, ausgewählt aus der Gruppe, bestehend aus Formel (Ib) und Formel (IIb) oder einer pharmazeutisch verträglichen Salz- oder Esterform davon: 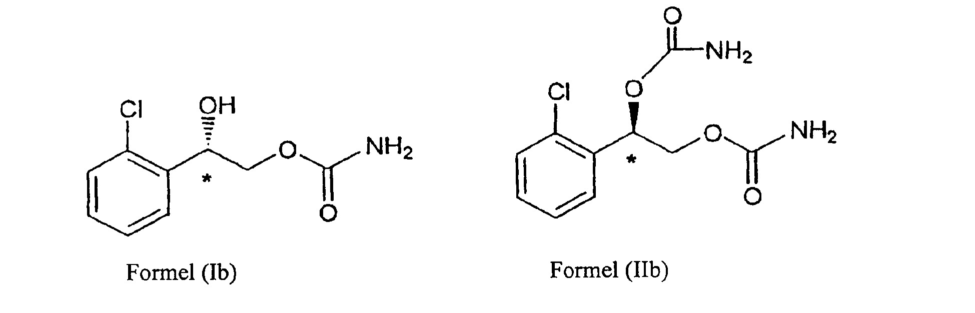 und wenigstens eine zusätzliche psychoaktive Medikation zur Verwendung in der Cotherapiebehandlung disruptiver Verhaltensstörungen.

15. Das Enantiomer nach Anspruch 14, wobei die Verbindung ein Enantiomer von Formel Ib ist, das bis zum Umfang von etwa 98% oder mehr überwiegt.

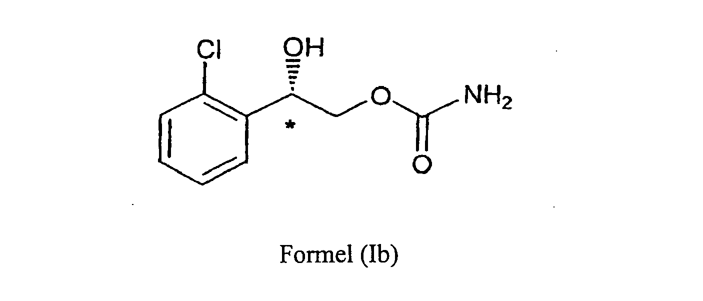

16. Das Enantiomer nach Anspruch 14, wobei die zusätzliche psychoaktive Medikation ausgewählt ist aus der Gruppe, bestehend aus: Stimulantien, einschließlich Methylphenidat, Amphetamin und Modafinil, Tranquilizern, wie etwa Molindon, Haloperidol und Chlorpromazin, Beruhigungsmitteln, einschließlich Benzodiazepinen, Antidepressiva, einschließlich, aber nicht beschränkt auf, tricyclischen Verbindungen, wie etwa Imipramin, Amitriptylin, Desipramin, Nortriptylin, Doxepin, Protriptylin, Trimipramin, Clomipramin, Amoxapin und dergleichen; tetracyclischen Verbindungen, wie etwa Maprotilin und dergleichen; nicht-cyclischen Verbindungen, wie etwa Nomifensin und dergleichen; Triazolopyridinen, wie etwa Trazodon und dergleichen; Serotonin-reuptake-Hemmern, wie etwa Fluoxetin, Sertralin, Paroxetin, Citalopram, Fluvoxamin und dergleichen; Serotoninrezeptorantagonisten, wie etwa Nefazodon und dergleichen; kombinierten Serotonin-noradrenergen-reuptake-Hemmern, wie etwa Venlafaxin, Milnacipran und dergleichen; noradrenergen und spezifischen serotonergen Mitteln, wie etwa Mirtazapin und dergleichen; Noradrenalin-reuptake-Hemmern, wie etwa Reboxetin und dergleichen; atypischen Antidepressiva, wie etwa Bupropion und dergleichen; natürlichen Produkten, wie etwa Kava-Kava, Johanniskraut und dergleichen; Nahrungsergänzungsmitteln, wie etwa s-Adenosylmethionin und dergleichen, Monoaminoxidasehemmern, wie etwa Phenelzin, Tranylcypromin, Moclobemid.

17. Das Enantiomer nach Anspruch 14, wobei die zusätzliche psychoaktive Medikation ausgewählt ist aus der Gruppe, bestehend aus: Methylphenidat, Amphetamin und Modafinil, Molindon, Haloperidol und Chlorpromazin, Fluoxetin, Sertralin, Paroxetin, Citalopram, Fluvoxamin, Nefazodon, Venlafaxin, Milnacipran, Mirtazapin, Bupropion.

## Revendications

1. Composé de la Formule 1 ou de la Formule 2: 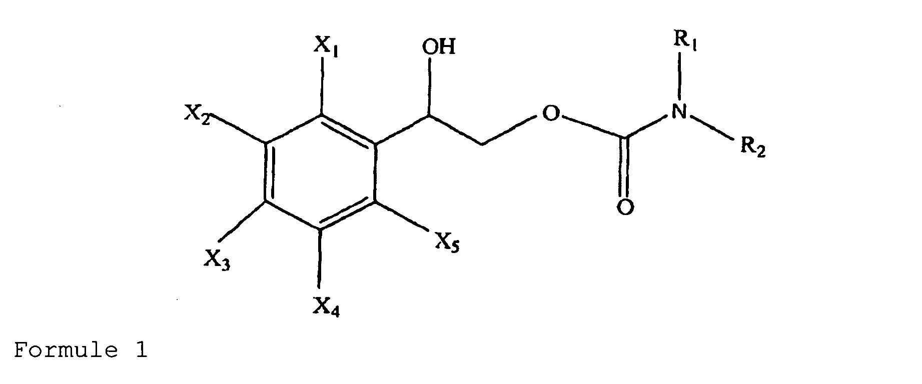 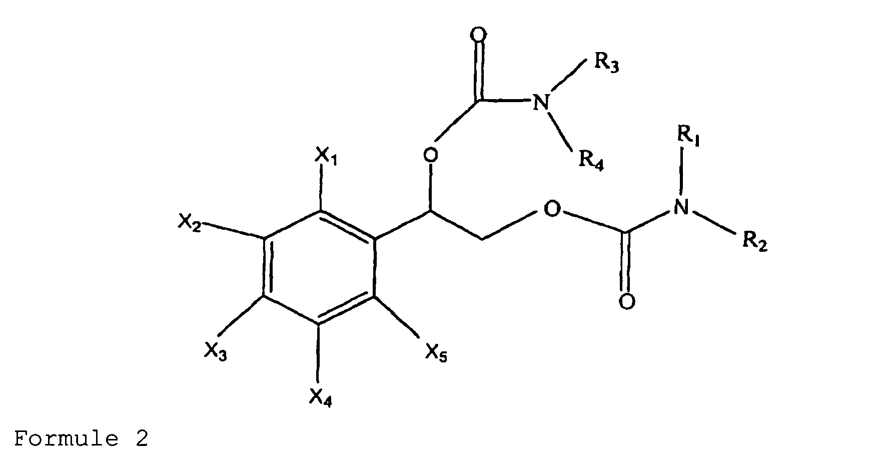 ou une forme de sel ou d'ester pharmaceutiquement acceptable de celui-ci pour utilisation dans le traitement de Troubles du Comportement Perturbateurs ou (DBDs)
où
R₁, R₂, R₃ et R₄ sont indépendamment hydrogène ou alkyleC₁-C₄,
où
alkyleC₁-C₄ est substitué ou non substitué par phényle, et où
phényle est substitué ou non substitué par jusqu'à cinq substituants indépendamment sélectionnés parmi:
halogène, alkyleC₁-C₄, alcoxyC₁-C₄, nitro, cyano et amino
où amino est optionnellement mono- ou disubstitué par alkyleC₁-C₄,
et X₁, X₂, X₃, X₄ et X₅ sont indépendamment hydrogène, fluor, chlore, brome ou iode.

2. Enantiomère, ou un sel ou ester pharmaceutiquement acceptable de celui-ci d'un composé de la revendication 1, sélectionné dans le groupe consistant en Formule (I) et Formule (II) ou un mélange énantiomérique dans lequel un énantiomère sélectionné dans le groupe consistant en Formule (I) et Formule (II) prédomine pour l'utilisation selon la revendication 1: 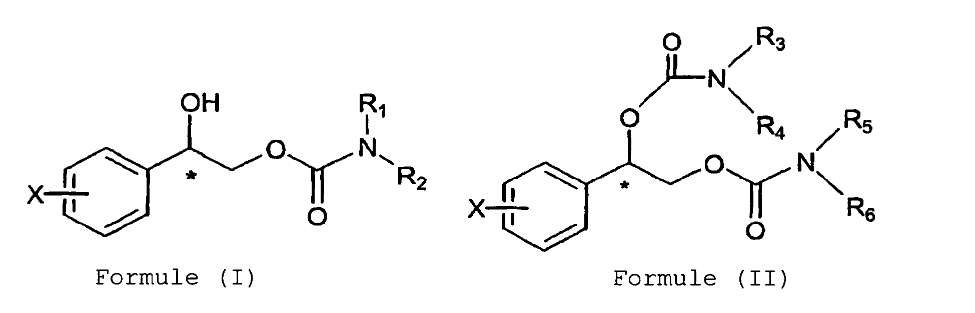 où
phényle est substitué à X par un à cinq atomes d'halogène sélectionnés dans le groupe consistant en fluor, chlore, brome et iode; et
R₁, R₂, R₃, R₄, R₅ et R₆ sont indépendamment sélectionnés dans le groupe consistant en hydrogène et alkyleC₁-C₄; où alkyleC₁-C₄ est optionnellement substitué par phényle (où phényle est optionnellement substitué par des substituants indépendamment sélectionnés dans le groupe consistant en halogène, alkyleC₁-C₄, alcoxyC₁-C₄, amino, nitro et cyano.

3. Enantiomère ou mélange énantiomérique selon la revendication 2, dans lequel l'énantiomère sélectionné dans le groupe consistant en Formule (I) et Formule (II) est un énantiomère sélectionné dans le groupe consistant en Formule (Ia) et Formule (IIa): 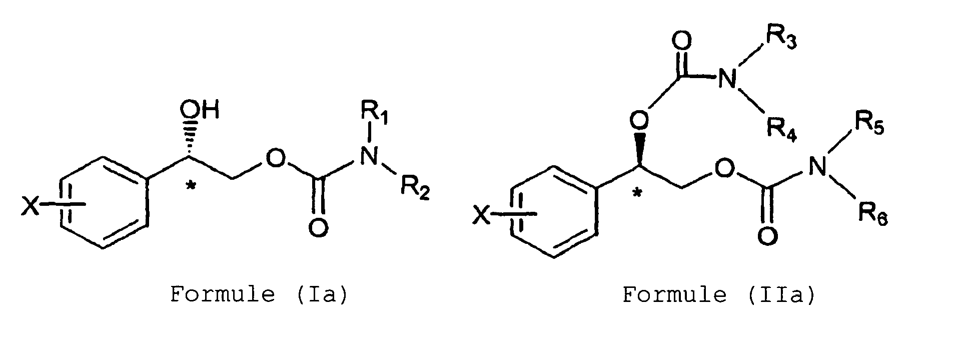 où
phényle est substitué à X par un à cinq atomes d'halogène sélectionnés dans le groupe consistant en fluor, chlore, brome et iode; et
R₁, R₂, R₃, R₄, R₅ et R₆ sont indépendamment sélectionnés dans le groupe consistant en hydrogène et alkyleC₁-C₄, où alkyleC₁-C₄ est optionnellement substitué par phényle, où phényle est optionnellement substitué par des substituants indépendamment sélectionnés dans le groupe consistant en halogène, alkyleC₁-C₄, alcoxyC₁-C₄, amino, nitro et cyano.

4. Composé, énantiomère ou mélange énantiomérique selon l'une quelconque des revendications 1, 2 ou 3, où X est chlore substitué à la position ortho du cycle phényle, et où R₁, R₂, R₃, R₄, R₅ et R₆ sont sélectionnés parmi hydrogène.

5. Enantiomère ou mélange énantiomérique selon la revendication 2, où l'énantiomère sélectionné dans le groupe consistant en Formule (I) et Formule (II) est un énantiomère sélectionné dans le groupe consistant en Formule (Ib) et Formule (IIb) ou une forme de sel ou d'ester pharmaceutiquement acceptable de celui-ci:

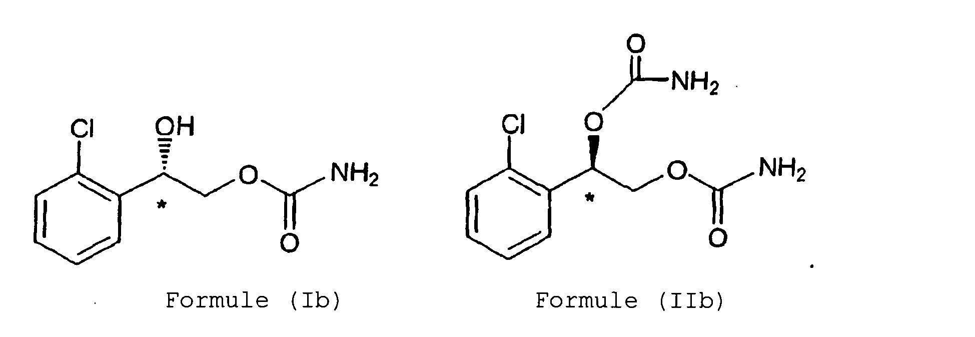

6. Enantiomère ou mélange énantiomérique selon l'une quelconque des revendications 2, 3 et 5, où un énantiomère sélectionné dans le groupe consistant en Formule (I) et Formule (II), Formule (Ia) et Formule (IIa) et Formule (Ib) et Formule (IIb) prédomine selon l'étendue d'environ 90% ou plus.

7. Enantiomère ou mélange énantiomérique selon l'une quelconque des revendications 2, 3 et 5, où un énantiomère sélectionné dans le groupe consistant soit en Formule (I) et Formule (II) soit en Formule (Ia) et Formule (IIa), soit en Formule (Ib) et Formule (IIb) prédomine selon l'étendue d'environ 98% ou plus.

8. Enantiomère ou mélange énantiomérique selon la revendication 5, où l'énantiomère est la Formule (Ib) et prédomine selon l'étendue de 98% ou plus.

9. Enantiomère ou mélange énantiomérique selon la revendication 5, où l'énantiomère est la Formule (IIb) et prédomine selon l'étendue de 98% ou plus.

10. Enantiomère du composé de la revendication 1 de la formule (Ib), qui prédomine selon l'étendue d'environ 98% ou plus pour l'utilisation selon la revendication 1:

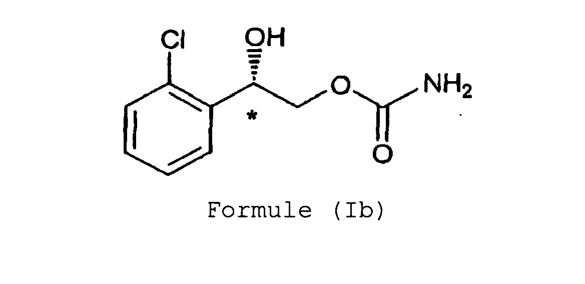

11. Composé selon la revendication 1 ou l'énantiomère selon la revendication 10, où le Trouble du Comportement Perturbateur est un Trouble de Conduite.

12. Composé selon la revendication 1 ou l'énantiomère selon la revendication 10, où le Trouble du Comportement Perturbateur est un Trouble Oppositionnel de Défience.

13. Composé selon la revendication 1, où le Trouble du Comportement Perturbateur est un Trouble du Comportement Perturbateur Non Spécifié Autrement.

14. Enantiomère du composé de la revendication 1 sélectionné dans le groupe consistant en Fomule (Ib) et Formule (IIb) ou une forme de sel ou d'ester pharmaceutiquement acceptable de celui-ci: 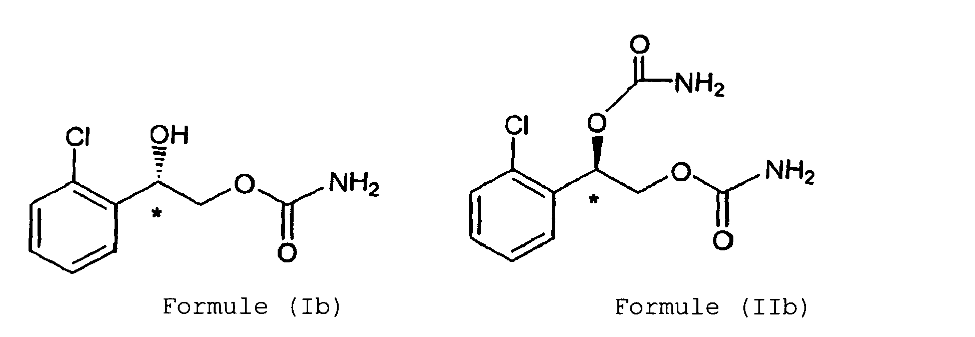 et au moins un médicament psychoactif additionnel pour utilisation dans le traitement de cothérapie des Troubles du Comportement Perturbateurs.

15. Enantiomère selon la revendication 14, où le composé est un énantiomère de la Formule Ib qui prédomine selon l'étendue d'environ 98% ou plus.

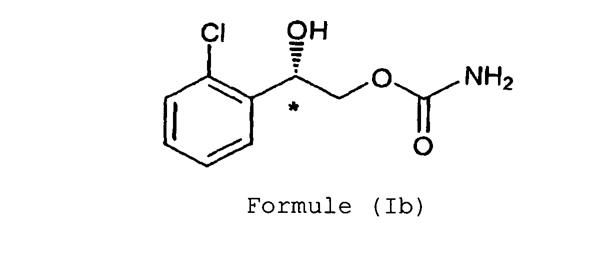

16. Enantiomère selon la revendication 14, dans lequel le médicament psychoactif additionnel est sélectionné dans le groupe consistant en: stimulants, incluant le méthylphénidate, amphétamine et modafinil, des tranquilisants majeurs, comme molindone, halopéridol et chlorpromazine, des tranquilisants mineurs, incluant des benzodiazépines, antidépresseurs, incluant mais n'étant pas limités à des tricycliques comme imipramine, amitriptyline, désipramine, nortriptyline, doxépine, protriptyline, trimipramine, clomipramine, amoxapine et analogue; des tétracycliques comme maprotiline et analogue; des non-cycliques comme nomifensine, et analogue; des triazolopyridines, comme trazodone et analogue; des inhibiteurs du recaptage de la sérotonine, comme fluoxétine, sertraline, paroxétine, citaloprame, fluvoxamine et analogue; des antagonistes du récepteur de la sérotonine comme néfazodone, et analogue; des inhibiteurs combinés du recaptage de sérotonine-noradrénergiques comme venlafaxine, milnaciprane et analogue; des agents sérotonergiques noradrénergiques et spécifiques comme mirtazapine et analogue; des inhibiteurs du recaptage de la noradrélanine comme réboxétine et analogue; des antidépresseurs atypiques comme bupropion et analogue; des produits naturels comme Kava-Kava, St. John's Wort, et analogue; des suppléments diététiques comme s-adénosylméthionine et analogue, des inhibiteurs de la mono-amine oxydase comme phénelzine, tranylcypromine, moclobemide.

17. Enantiomère selon la revendication 14, dans lequel le médicament pyschoactif additionnel est sélectionné dans le groupe consistant en: méthylphénidate, amphétamine et modafinil, molindone, halopéridol et chlorpromazine, fluoxétine, sertraline, paroxétine, citaloprame, fluvoxamine, néfazodone, venlafaxine, miinaciprane, mirtazapine, bupropione.
